(19) Europäisches Patentamt
European Patent Office
Office européen des brevets

(11) Veröffentlichungsnummer: **0 270 992 B1**

(12) # EUROPÄISCHE PATENTSCHRIFT

(45) Veröffentlichungstag der Patentschrift: **08.06.94**

(51) Int. Cl.$^5$: **C07H 15/252**, A61K 31/70

(21) Anmeldenummer: **87117853.9**

(22) Anmeldetag: **03.12.87**

(54) **Zytostatisch wirksame Anthracyclinderivate.**

(30) Priorität: **08.12.86 DE 3641833**

(43) Veröffentlichungstag der Anmeldung:
**15.06.88 Patentblatt 88/24**

(45) Bekanntmachung des Hinweises auf die Patenterteilung:
**08.06.94 Patentblatt 94/23**

(84) Benannte Vertragsstaaten:
**AT BE CH DE ES FR GB GR IT LI LU NL SE**

(56) Entgegenhaltungen:
**EP-A- 0 022 574**
**EP-A- 0 063 776**

**CHEMICAL ABSTRACTS, Band 92, Nr. 17, 28 April 1980, Columbus, OH (US); Nr. 147142f&NUM;**

**CHEMICAL ABSTRACTS, Band 94, Nr. 15, 13 April 1981, Columbus, OH (US); M.J. EGORIN et al., Seite 11, Nr. 114172d&NUM;**

**JOURNAL OF ANTIBIOTICS, Band XXXII, Nr. 8, August 1979; T. OKI et al., Seiten 801-819&NUM;**

(73) Patentinhaber: **BEHRINGWERKE Aktiengesellschaft**
**Postfach 1140**
**D-35001 Marburg(DE)**

(72) Erfinder: **Hermentin, Peter, Dr.**
**Barfüssertor 30**
**D-3550 Marburg(DE)**
Erfinder: **Paal, Michael, Dr.**
**Eppendorfer Landstrasse 6B**
**D-2000 Hamburg 20(DE)**
Erfinder: **Kraemer, Hans Peter, Dr.**
**Birkenweg 16**
**D-3550 Marburg(DE)**
Erfinder: **Kolar, Cenek, Dr.**
**Deutschhausstrasse 20**
**D-3550 Marburg(DE)**
Erfinder: **Hoffmann, Dieter, Dr.**
**Im Stetefeld 6**
**D-3551 Lahntal(DE)**
Erfinder: **Gerken, Manfred, Dr.**
**Wannkopfstrasse 12**
**D-3550 Marburg(DE)**
Erfinder: **Berscheid, Hans Gerd, Dr.**
**Rheinlandstrasse 21**
**D-6231 Schwalbach am Taunus(DE)**

**EP 0 270 992 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**JOURNAL OF ANTIBIOTICS, Band 36, Nr. 8, August 1983, Tokyo (JP); T. UCHIDA et al., Seiten 1080-1083&NUM;**

Erfinder: **Böttger, Dirk, Dr.**
**Bahnstrasse 4**
**D-6237 Liederbach(DE)**

## Beschreibung

Die Erfindung betrifft neue, zytostatisch wirksame Anthracyclinderivate, Verfahren zu ihrer Herstellung sowie ihre Verwendung in Arzneimitteln.

Die Substanzklasse der Anthracycline ist schon seit langem bekannt. Seit der Strukturermittelung der Rhodomycine, des Adriamycins bzw. des Daunomycins und dem Bekanntwerden der zytostatischen Wirksamkeit gewisser Vertreter dieser Anthracyclinklasse Wege aus Vertretern der Actinomyceten-Gattung Spreptomyces isoliert und in ihrer Wirkung erforscht.

Es ist bekannt, daß einige Anthracycline, wie z.B. Adriamycin, Daunomycin, Aclacinomycin, 4'-epi-Adriamycin, 4'-Methoxyadriamycin oder 4'-Desoxyadriamycin bereits für die Therapie von Tumoren verwendet werden. Diesen Verbindungen ist gemeinsam, daß sie in der Position 7 des jeweiligen Aglycons entweder den Zucker α-L-Daunosamin (z.B. in den Anthracyclinen Adriamycin, Daunomaycin und 4-Demethoxyadriamycin) oder den Zucker -α-L-Rhodosamin (z.B. in den Anthracyclinen N,N-Dimethyladaunomycin, Aklavin oder β-Rhodomycin I) tragen. Ferner sind Anthracycline bekannt, welche in Position 7 des jeweiligen Aglycons einen an der 3'-Aminogruppe substituierten α-L-Daunosamin-Baustein tragen (z.B. in den Anthracyclinen N-Trifluoracetyladriamycin, N-Benzyladricamycin, N,N-Dibenzyladriamycin, Morpholinadaunomycin, Cyanomorpholinodaunomycin oder Cyanomorpholinoadriamycin).

Bekannt sind weiterhin Anthracycline, welche in der Position 7 des Aglycons eine Trisaccharid-Einheit aufweisen, wie beispielsweise Aclacinomycin A, sowie Anthracycline welche neben einer Trisaccharid-Einheit in der Position 7 des Aglycons auch eine Monosaccharid- oder Trisaccharid-Einheit in Position 10 des Aglycons aufweisen, wie beispielsweise Cytorhodin S oder Cytorhodin P.

Diesen Verbindungen ist gemeinsam, daß die Zuckereinheit, welche in der Position 7 oder der Position 10 des Aglycons gebunden ist, stets α-L-Rhodosamin ist.

Aus Chemical Abstracts, Band 92, Nr. 17, Ref. Nr. 147142f sind ferner die Verbindungen 3'-N-Methyldaunomycin und 3'-N-Methyladriamycin bekannt.

Aus der US PS 4 591 637 sind schließlich noch Verbindungen der nachfolgenden Formel I bekannt, in denen $R^1$ = H, $R^2$ = H,OH oder-$OCH_3$,$R^3$ = $R^4$ = OH, $R^5$ = H, $R^6$ = Äthyl,Methylcarbonyl, Hydroxymethylcarbonyl, Alkylhydroxy oder Alkyldihydroxy, $R^7$ = H und $R^8$ eine Cyanomethylgruppe ist.

Bei der tumortherapeutischen Verwendung dieser bekannten Anthracycline liegt ein wesentliches Problem darin, daß sie neben der gewünschten zytostatischen Wirksamkeit unerwünschte Nebenwirkungen aufweisen, wie beispielsweise eine hämatologische oder cardiale Toxizität.

Ausgehend von diesem Stand der Technik ist es Aufgabe der vorliegenden Erfindung, neue, Anthracyclinderivate zu schaffen, die möglichst nicht kreuzresistent gegenüber Adriamycin sind und die sich durch ein neues Wirkungsspektrum und eine im Vergleich zu Adriamiycin geringere Toxizität auszeichnen und somit vorteilhafte Anwendung in der Tumortherapie finden können.

Gelöst wird diese erfindungsgemäße Aufgabe mit neuen zytostatisch wirksamen Anthracyclinderivaten, die der nachfolgenden allgemeinen Formel (I)

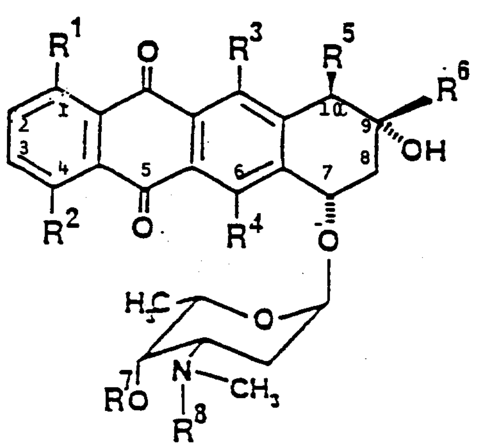

I

entsprechen, welche gegebenenfalls als Salz einer anorganischen oder organischen Säure vorliegen, und in der die Reste folgende Bedeutung haben:

$R^1$ ist Wasserstoff oder eine Hydroxygruppe,

$R^2$ ist Wasserstoff, eine Hydroxy- -oder eine Methoxygruppe,

$R^3$ ist Wasserstoff oder eine Hydroxygruppe,
$R^4$ ist Wasserstoff oder eine Hydroxygruppe,
$R^5$ ist Wasserstoff, eine Hydroxy-, eine Methoxycarbonylgruppe oder ein Rest der allgemeinen Formel II

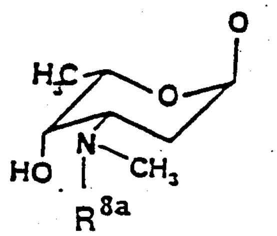

II

in welcher $R^{8a}$ die unter $R^8$ angegebene Bedeutung hat, oder ein Rest der Formel III,

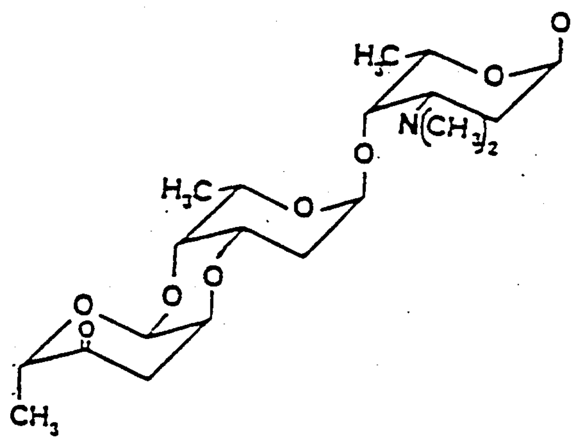

III

$R^6$ ist Äthyl, Methylcarbonyl, Hydroxymethylcarbonyl, Alkylhydroxy oder Alkyldihydroxy,
$R^7$ ist Wasserstoff,
$R^8$ ist Wasserstoff oder eine Cyanomethylgruppe oder ein Substituent der allgemeinen Formel $COR^9$ oder $CH_2R^{10}$,wobei $R^9$ Wasserstoff, $CH_3$, $CF_3$ oder $CCl_3$ und
$R^{10}$ $C_1$- bis $C_8$-Alkyl, substituiertes Alkyl, Phenyl oder substituiertes Phenyl ist, ausgenommen die Verbindungen der Formel I, in welcher
$R^1$ = H, $R^2$ = OH, $R^3$ = H, $R^4$ = OH, $R^5$ = $COOCH_3$,
$R^6$ = $CH_2CH_3$ und $R^8$ = H ist;
$R^1$ = H, $R^2$ = $OCH_3$, $R^3$ = $R^4$ = OH, $R^5$ = H, $R^6$ = $COCH_3$ und $R_8$ = H sowie $R^1$ = H, $R^2$ = $OCH_3$, $R^3$ = $R^4$ = OH, $R^5$ = H, $R^6$ = $COCH_2OH$ und $R^8$ = H sind,
sowie weiterhin für den Fall, daß $R^8$ eine Cyanomethylgruppe ist, diejenigen Verbindungen, in welchen $R^1$ = H ist, $R^2$ die genannte Bedeutung hat, $R^3$ = OH, $R^4$ = OH, $R^5$ = H ist, $R^6$ die genannte Bedeutung hat und $R^7$ = H ist.

Die Substituenten am Phenylrest können dabei in ortho-, meta- oder para-Stellung vorliegen und als Beispiele können genannt werden: Methyl, Äthyl, Hydroxy, Methoxy, Äthoxy, Nitro, Cyano, Fluor, Chlor oder Brom.

Bei besonders bevorzugten Aglyconderivaten der vorliegenden Erfindung weisen die Substituenten in der obengenannten Formel die folgende Bedeutung auf:
$R^1$ bis $R^4$ und $R^6$ wie vorstehend angegeben, $R^5$ Wasserstoff, eine Hydroxy oder eine Methoxycarbonylgruppe, $R^7$ Wasserstoff und $R^8$ Wasserstoff, Cyanomethyl oder ein Substituent der allgemeinen Formel $COR^9$ mit $R^9$ gleich H, $CH_3$, $CF_3$ oder $CCl_3$ oder ein Substituent der allgemeinen Formel $CH_2R^{10}$, wobei $R^{10}$ $C_1$- bis $C_8$-Alkyl, substituiertes Alkyl, Phenyl oder substituiertes Phenyl ist, das in ortho-, meta- oder para-Stellung durch Methyl, Äthyl, Hydroxy, Methoxy, Äthoxy, Nitro, Cyano, Fluor, Chlor oder Brom substituiert ist.

Weitere besonders bevorzugte Anthracycinderivate sind die folgenden, die gegebenenfalls auch als Säureadditionssalze von physiologisch unbedenklichen anorganischen oder organischen Säuren vorliegen

können:
Anthracyclinderivate der Formel I, worin $R^1$ = H, $R^2$ = $R^3$ = $R^4$ = $R^5$ = OH und $R^6$ = $CH_2CH_3$ ist;
Anthracyclinderivate der Formel I, worin $R^1$ = $R^2$ = $R^3$ = $R^4$ = $R^5$ = OH und $R^6$ = $CH_2CH_3$ ist;
Anthracyclinderivate der Formel I, worin $R^1$ = H, $R^2$ = $OCH_3$, $R^3$ = $R^4$ = OH, $R^5$ = H und $R^6$ = $COCH_3$ ist;
Anthracyclinderivate der Formel I, worin $R^1$ = H, $R^2$ = $OCH_3$, $R^3$ = $R^4$ = OH, $R^5$ = H und $R^6$ = $COCH_2OH$ ist;
Anthracyclinderivate der Formel I, worin $R^1$ = H, $R^2$ = $OCH_3$, $R^3$ = $R^4$ = OH, $R^5$ = H und $R^6$ = CH(OH)-$CH_3$ ist;
Anthracyclinderivate der Formel I, worin $R^1$ = H, $R^2$ = $OCH_3$, $R^3$ = $R^4$ = OH, $R^5$ = H und $R^6$ = CH(OH)-$CH_2OH$ ist;
Anthracyclinderivate der Formel I, worin $R^1$ = $R^2$ = $R^3$ = $R^4$ = OH, $R^5$ = $COOCH_3$ und $R^6$ = $CH_2CH_3$ ist;
Anthracyclinderivate der Formel I, worin $R^1$ = H, $R^2$ = $R^3$ = $R^4$ = OH, $R^5$ = $COOCH_3$ und $R^6$ = $CH_2CH_3$ ist;
Anthracyclinderivate der Formel I, worin $R^1$ = H, $R^2$ = OH, $R^3$ = H, $R^4$ = OH, $R^5$ = $COOCH_3$, $R^6$ = $CH_2CH_3$ und $R^8$ ungleich $R^7$ ist;
Anthracyclinderivate der Formel I, worin $R^5$ ein Rest der Formel II ist und $R^8$ und $R^{8a}$ identisch sind;
Anthracyclinderivate der Formel I, worin $R^5$ ein Rest der Formel II ist und $R^8$ und $R^{8a}$ identisch und $R^1$ = H oder OH, $R^2$ = $R^3$ = $R^4$ = OH und $R^6$ = $CH_2CH_3$ sind;
Anthracyclinderivate der Formel I, worin $R^5$ ein Rest der Formel III ist;
Anthracyclinderivate der Formel I, worin $R^5$ ein Rest der Formel III und $R^1$ = H oder OH, $R^2$ = $R^3$ = $R^4$ = OH und $R^6$ = $CH_2CH_3$ sind;
Das erfindungsgemäße Verfahren zur Herstellung der neuen zytostatisch wirksamen Anthracyclinderivate geht aus von auf biologischem Wege erhältlichen Anthracyclinverbindungen (T.Oki in Antracycline Antibiotics, H.S.E1 Khadem,ed.,Academic Press 1982, S.75), die entweder direkt in Form des Rhodosaminderivats vorliegen oder durch N,N-Dimethylierung des korrespondierenden Daunosaminyl-Anthracyclinons (Tong et al.,J.Med.Chem. (1979),22,912), oder aber durch Umsetzung der entsprechenden Anthracyclinonverbindung mit Rhodosamin in einfacher Weise erhalten werden Die Verknüpfung zwischen einem auf biologischem Wege erhaltenen Anthracyclinon und dem Rhodosamin kann dabei gem.Verfahren von Essery und Doyle (Can.J. Chem. (1980) 58, 1869 erfolgen. Dabei wird der Rhodosaminylrest in der 7-Stellung des Anthracyclinons gebunden.

Es ist an sich bereits bekannt, daß Daunomycin (7-O-α-L-Daunosaminyl-daunomycinon) oder Adriamycin. (7-O-α-L-Daunosaminyl-adriamycinon) unter Lichteinwirkung photolytisch zersetzt werden, (Tavoloni et al., J.Pharmacol.(1980), 32 860); wobei sich offenbar Polymere bilden
(Williams & Tritton, Photochem. Photobiol. (1981), 34, 131).

Andererseits ist es bekannt, daß auch Aclacinomycin A, ein Anthracyclin, in welchem in Position 7 des Aglycons Aklavinon das Trisaccharid [L-Cinerulose A]-α-(1 → 4)-[2-Desoxy-L-fucose]-α(1 → 4)-[α-L-Rhodosamin] gebunden ist, ebenfalls in geringem Umfang einer photolytischen Zersetzung unterliegt. Als Zersetzungsprodukte wurden dabei in gezielten Ansätzen das 3'-N-monodemethylierte Aklacinomycin A in 3,5% Ausbeute und das 3'-N-didemethylierte Aklacinomycin A in 5,5% Ausbeute isoliert, wobei im ersten Fall Äthyläther und im letzten Fall Chloroform als Lösungsmittel verwendet wurde. (Oki et al., J. Antibiotics (1979), 32, 801).

Durch milde Säurehydrolyse gelang anschließend der Zugang zu 3'-N-Methyl-daunosaminyl-aklavinon, der einzigen bislang beschriebenen Verbindung der Formel I, in welcher $R^1$ = H, $R^2$ = OH, $R^3$ = H, $R^4$ = OH, $R^5$ = $COOCH_3$, $R^6$ = $CH_2CH_3$ und $R^7$ = $R^8$ = H ist.

Es ist ferner aus Tanaka et al., J.Antibiotics (1982),35,312, bekannt, daß auch das Anthracyclin, in welchem die vorstehend genannte Trisaccharid-Kette in Position 7 des Aglycons Daunomycinon gebunden ist, einer photolytischen Zersetzung durch Sonnenlicht unterliegt. Als Zersetzungsprodukte wurden in geringen Ausbeuten 3'-N-monodemethyliertes Ahthracyclin (in 15% Ausbeute) und das 3'-N-didemethylierte Anthracyclin (in 8 % Ausbeute) isoliert, wobei die photolytische Zersetzungsreaktion in Chloroform als Lösungsmittel durchgeführt wurde.

In EP-A-0 063 776 ist beschrieben, daß die korrespondierenden Adriamycin- und Carminomycinontriglycoside in analoger Weise durch Einwirkung von Sonnenlicht in geringer Ausbeute zu den korrespondierenden 3'-N-monodemethylierten Anthracyclinen zersetzt werden.

Überraschenderweise wurde nunmehr gefunden, daß sich 7-O-α-L-Rhodosaminyl-anthracyclinon-Verbindungen der Formel I, in welcher $R^1$ bis $R^4$ und $R^6$ die genannte Bedeutung haben, $R^5$ Wasserstoff oder eine Hydroxy- oder eine Methoxycarbonylgruppe ist, $R^7$ Wasserstoff und $R^8$ eine Methylgruppe ist, im

Gegensatz zu den literaturbekannten Feststellungen, gezielt und in guter Ausbeute in ihre korrespondierenden 3'-N-monodemethylierten Analoga überführen lassen, ohne daß in nennenswertem Umfang eine Didemethylierung beobachtet wird.

Hierdurch eröffnet sich ein guter und einfacher Zugang zu der neuen Verbindungsklasse der 7-O-(3'-N-Methyl-α-L-daunosaminyl)-anthracyclinone, welche auf dem Wege der reduktiven Alkylierung mit Formaldehyd und Natriumcyanoborhydrid, wie am Beispiel von Daunomycin und Adriamycin gezeigt wurde (Tong et al., J.Med. Chem. (1979), 22, 912), nicht zugänglich sind.

Weiterhin wurde überraschenderweise festgestellt, daß auch Anthracycline, welche in der Position 10 des Aglycons einen α-L-Rhodosaminyl-Rest tragen und in Position 7 einen α-L-Rhodosaminyl-Rest, in welchem R ein Rest der allgemeinen Formel IV ist, gezielt und in guter Ausbeute zu den korrespondierenden Anthracyclinen demethyliert werden können, welche in Position 10 des Aglycons einen 3'-N-Methyl-α-L-daunosaminyl-Rest tragen.

Weiterhin wurde festgestellt, daß Anthracycline, welche sowohl in Position 7 als auch in Position 10 des Aglycons den Zuckerrest Rhodosamin aufweisen, gezielt und in guter Ausbeute zu den entsprechenden Anthracyclinen demethyliert werden können, welche sowohl in Position 7 als auch in Position 10 des Aglycons den Rest 3'-N-Methyl-α-L-daunosamin aufweisen.

Schließlich wurde auch bei diesen Untersuchungen festgestellt, daß Anthracycline, welche in Position 7 des Aglycons den Zucker Rhodosamin tragen und in welchen $R^5$ (in Position 10 des Aglycons) eine Struktur der Formel III ist, gezielt und ebenfalls in guter Ausbeute zu den korrespondierenden Anthracyclinen demethyliert werden können, welche in Position 7 des Aglycons den Rest 3'-N-Methyl-α-L-daunosamin tragen.

Ferner wurde gefunden, daß sich alle diese durch photolytische Demethylierung von Rhodosaminyl-Anthracyclinonen gewonnenen 3'-N-Methyl-daunosaminyl-anthracyclinone in einfacher und an sich bekannter Weise an ihrer Methylamino-Funktion selektiv modifizieren bzw. substituieren lassen, wodurch zahlreiche weitere neue zytostatisch wirksame Anthracycline erhalten werden.

Überraschenderweise wurde auch gefunden, daß die photolytische Demethylierung von 7-O-α-L-Rhodosaminyl-anthracyclinonen in einer Nebenreaktion zu Verbindungen der Formel I führen kann, in welcher $R^1$ bis $R^4$ und $R^6$ die angegebene Bedeutung haben, $R^5$ Wasserstoff oder eine Hydroxy- oder Methoxycarbonylgruppe, $R^7$ Wasserstoff und $R^8$ eine Formylgruppe ist.

Ausgehend von diesen Feststellungen ist das erfindungsgemäße Verfahren zur Herstellung der neuen zytostatisch wirksamen Anthracyclinderivate der vorliegenden Erfindung dadurch gekennzeichnet, daß bei einer Verbindung der Formel I, in der die Reste folgende Bedeutung haben:

- $R^1$ ist Wasserstoff oder eine Hydroxygruppe,
- $R^2$ ist Wasserstoff, eine Hydroxy- oder Methoxygruppe,
- $R^3$ ist Wasserstoff oder eine Hydroxygruppe,
- $R^4$ ist Wasserstoff oder eine Hydroxygruppe,
- $R^5$ ist Wasserstoff, eine Hydroxy-, eine Methoxycarbonylgruppe oder ein Rest der allgemeinen Formel II, in welcher $R^{8a}$ die unter $R^8$ angegebene Bedeutung hat, oder eine Struktur der Formel III,
- $R^6$ ist Äthyl, Methylcarbonyl, Hydroxymethylcarbonyl, Alkylhydroxy oder Alkyldihydroxy,
- $R^7$ ist Wasserstoff und
- $R^8$ ist ein Methylrest,

  a) auf photolytischem Wege in Gegenwart eines Lösungsmittelgemisches aus halogeniertem Alkan und einem Alkohol unter Lichteinwirkung eine am Stickstoff gebundenen Methylgruppe abgespalten wird und

  b) gegebenenfalls das in Stufe a) gebildete 3'-N-Methylaminoderivat mit aktiviertem Acetonitril oder gegebenenfalls

  c) das in Stufe a) gebildete 3'-N-Methylaminoderivat mit einer aktivierten Acylverbindung oder gegebenenfalls

  d) das in Stufe a) gebildete 3'-N-Methylaminoderivat mit einem Aldehyd oder gegebenenfalls

  e) das in Stufe a) gebildete 3'-N-Methylaminoderivat mit einer gegebenenfalls substituierten aliphatischen Jod-, Tosyl- oder Trifluormethansulfonylverbindung umgesetzt wird und gegebenenfalls

  f) das Reaktionsprodukt der Stufe a), b), c), d) oder e) in an sich bekannter Weise in das Salz einer anorganischen oder organischen Säure überführt wird.

Es hat sich insbesondere im Hinblick auf die erzielte Ausbeute als besonders vorteilhaft erwiesen, wenn die photolytische Abspaltung der Methylgruppe in Gegenwart eines Lösungsmittelgemisches aus 10 bis 30 Volumenteilen Chloroform und 1 Volumenteil Alkohol in einer Konzentration zwischen 0,1 und 1 Millimol pro

Liter bei einer Temperatur zwischen 0°C und der Siedetemperatur des Lösungsmittelgemisches unter starker Lichteinwirkung durchgeführt wird. Gute Ergebnisse werden auch unter Verwendung von Tetrachlorkohlenstoff anstelle von Chloroform erhalten. Besonders gute Ergebnisse werden erzielt, wenn als Alkohol Methylalkohol Verwendung findet.

Für die photolytische Demethylierungsreaktion erweist es sich als besonders vorteilhaft, wenn das Lösungsmittelgemisch mit der darin enthaltenen Anthracyclin-Verbindung eine möglichst große Oberfläche aufweist, die beispielsweise dadurch erzielt werden kann, daß die Lösung in breitwannige Gefäße oder Rieselapparaturen gefüllt wird, um dann mit Sonnenlicht oder einer künstlichen Lichtquelle, bevorzugt mit dem sichtbaren Licht einer Glühlampe, z.B. einer Photolampe oder einem 500 Watt-Strahler, aus einer geringen Entfernung von beispielsweise 20 bis 30 cm bestrahlt zu werden, wobei das Licht zur Erhöhung der photolytischen Ausbeute zweckmäßigerweise durch eine Aluminiumfolie reflektiert wird.

Man erhält bei dem erfindungsgemäßen photolytischen Verfahren eine Verbindung der Formel I, in welcher $R^1$ bis $R^7$ die obengenannte Bedeutung haben und $R^8$ Wasserstoff ist.

Die Verbindung wird durch Entfernung des Lösungsmittels isoliert und durch mehrmaliges Ausschütteln einer sauren wässrigen Lösung, z.B. einer wässrigen Salzsäure-haltigen Lösung von pH 2 bis 4, mit einem geeigneten organischen Lösungsmittel, beispielsweise mit Chloroform, extrahiert und die wässrige Phase zur Isolierung der entsprechenden Säureadditionsverbindung lyophilisiert oder die wässrige Phase z.B. mit Natriumhydrogencarbonat neutral oder basich, z.B. auf pH 8, gestellt und erneut mit einem geeigneten organischen Lösungsmittel, beispielsweise mit Chloroform, extrahiert, wobei die gebildete 3'N-Methylaminoverbindung in die organische Phase übergeht, aus welcher sie nach Entfernung des Lösungsmittels, gegebenenfalls nach weiteren, an sich bekannten Reinigungsverfahren, z.B. nach Kieselgel-Chromatographie, in reiner Form isoliert wird.

Die solchermaßen erhaltene Verbindung der Formel I, in welcher $R^1$ bis $R^7$ die obengenannte Bedeutung haben und $R^8$ Wasserstoff ist, kann in geeigneter und an sich bekannter Weise an der Methylaminogruppe weiter derivatisiert werden. Dies kann beispielsweise durch Umsetzung mit Jodacetonitril oder Bromacetonitril, in einem geeigneten Lösungsmittel, beispielsweise Dimethylformamid, in Gegenwart einer geeigneten Base, beispielsweise Triäthylamin, geschehen, worauf eine Verbindung der Formel I erhalten wird, in welcher

a) in dem Falle, daß $R^5$ in der Ausgangsverbindung Wasserstoff oder eine Hydroxy oder eine Methoxycarbonylgruppe und $R^7$ Wasserstoff war, $R^1$ bis $R^4$ und $R^6$ die genannte Bedeutung haben, $R^5$ Wasserstoff oder eine Hydroxy- oder eine Methoxycarbonylgruppe, $R^7$ Wasserstoff und $R^8$ Cyanomethylist oder

b) in dem Falle, daß $R^5$ in der Ausgangsverbindung ein $\alpha$-L-Rhodosaminyl-Rest und $R^7$ Wasserstoff war, $R^1$ bis $R^4$ und $R^6$ die genannte Bedeutung haben, $R^5$ eine Struktur der Formel II mit $R^{8a}$ = Cyanomethyl, $R^7$ Wasserstoff und $R^8$ wiederum Cyanomethyl ist oder

c) in dem Falle, daß $R^5$ in der Ausgangsverbindung ein Rest der Formel III und $R^7$ Wasserstoff war, $R^1$ bis $R^4$ und $R^6$ die genannte Bedeutung haben, $R^5$ eine Struktur der Formel III, $R^7$ Wasserstoff und $R^8$ Cyanomethyl ist.

Durch Umsetzung mit einem Acylierungsmittel, z.B. mit Acetanhydrid oder Acetylchlorid oder Trifluoracetanhydrid oder dem gemischten Anhydrid aus Ameisensäure und Essigsäure in einem geeigneten Lösungsmittel, z.B. in Methanol, wird eine Verbindung der Formel I erhalten, in welcher in den vorstehend genannten Fällen a) bis d) $R^8$ bzw. $R^{8a}$ statt der Cyanomethylgruppe ein Substituent der allgemeinen Formel $COR^9$ ist, wobei $R^9$ Wasserstoff, $CH_3$, $CF_3$ oder $CCl_3$ sein kann.

Durch Umsetzung mit einem aliphatischen oder aromatischen Aldehyd der Formel $R^{10}CHO$, der gegebenenfalls in geeigneter Weise substituiert sein kann, wobei der Substituent so beschaffen sein muß, daß er gegenüber der Aldehydfunktion inert ist, wie z.B. Methyl, Äthyl, Hydroxy, Methoxy, Äthoxy, Nitro, Cyano, Fluor, Chlor oder Brom, in Gegenwart eines geeigneten Reduktionsmittels, wie z.B. Natriumcyanoborhydrid, wird eine Verbindung der Formel I erhalten, in welcher in den vorstehend genannten Fällen a) bis d) $R^8$ bzw. $R^{8a}$ statt der Cyanomethylgruppe ein Substituent der allgemeinen Formel $CH_2R^{10}$ ist, wobei $R^{10}$ durch die Struktur des eingesetzten Aldehyds definiert ist.

Durch Umsetzung mit einer aliphatischen oder substituierten aliphatischen Jod-, Tosyl- oder TrifluormethansulfonylVerbindung, gegebenenfalls unter Zusatz einer geeigneten Base, z.B. Triäthylamin, wird eine Verbindung der Formel I erhalten, in welcher in den vorstehend genannten Fällen a) bis d) $R^8$ bzw. $R^{8a}$ statt der Cyanomethylgruppe ein Substituent der allgemeinen Formel $CH_2R^{10}$ ist, wobei $R^{10}$ durch die Struktur des eingesetzten Jodids, Tosylats oder Trifluormethansulfonats definiert ist.

Die nach dem erfindungsgemäßen Verfahren erhaltenen neuen Anthracyclinderivate zeichnen sich durch zytostatische Aktivität aus und sie können daher zusammen mit den üblichen pharmazeutischen Konfektionierungs- und/oder Verdünnungsmitteln zu Arzneimitteln verarbeitet werden, die in der Krebstherapie Anwendung finden. Die Dosierungs- und Anwendungsweise entspricht dabei im wesentlichen derjenigen für die bekannten Substanzen Adriamycin, Daunomycin, Aclacinomycin, 4'-epi-Adriamycin, 4'-Methoxyadriamycin oder 4'-Desoxyadriamycin.

Die solchermaßen hergestellten Arzneimittel können zusätzlich noch andere Wirkstoffe enthalten, sofern diese zusammen mit den erfindungsgemäßen Verbindungen keine unerwünschten Nebenwirkungen zeigen.

Die zytostatische Wirksamkeit der erfindungsgemäßen Verbindungen wurde anhand von L1210 Leukämiezellen der Maus getestet. Hierzu wurde die Koloniebildung von L1210 Leukämiezellen in Agarplatten herangezogen. Diese Methode dient zum Nachweis des Einflusses der Testsubstanzen auf das Wachstumsverhalten der Zellen über 1 Stunde oder über mehrere Generationen. Bei einer Zellzykluszeit von 10-12 Stunden werden dabei in der Testzeit von 7 Tagen etwa 14 aufeinanderfolgende Generationen beobachtet. Die erfindungsgemäßen zytostatisch wirksamen Substanzen bewirken in diesem Test eine Reduktion der zu beobachtenden Koloniezahl gegenüber einer unbehandelten Kontrollprobe.

Einzelheiten des Testverfahrens ergeben sich aus der nachfolgenden Verfahrensweise zur Ermittlung der Koloniebildung.

Verfahrensweise zur Ermittlung der Koloniebildung von L1210 Leukämiezellen in Soft-Agar.

500 Leukämiezellen pro Platte wurden mit unterschiedlichen Konzentrationen der Testsubstanz 1 Stunde bei 37 °C inkubiert. Anschließend wurden die Zellen zweimal mit McCoy5A-Medium gewaschen und schließlich in Petrischalen nach Zugabe von 0,3% Agar ausgegossen. Kontrollen wurden lediglich mit frischem Medium inkubiert. Anstelle der einstündigen Inkubation wurden in machen Fällen unterschiedliche Konzentrationen und Testsubstanzen der oberen Agarschicht zugemischt, um so eine kontinuierliche Exposition der Zellen über die gesamte Inkubationszeit zu erreichen. Nach Erstarren des Agars wurden die Platten im Brutschrank 7 Tage bei 37°C inkubiert (5 Vol.-% $CO_2$, 95% relative Luftfeuchtigkeit). Anschließend wurde die Zahl der entstandenen Kolonien mit einem Durchmesser von mehr als 60 µm gezählt. Die Ergebnisse wurden angegeben als Koloniezahl in behandelten Agarplatten in Prozent der unbehandelten Kontrolle. Aus der so erhaltenen Dosiswirkungskurve wurde die $IC_{50}$ als Maß für die Wirksamkeit der Substanz ermittelt. Die Ergebnisse für die hier beschriebenen Verbindungen im Vergleich zu Adriamycin sind in der nachfolgenden Tabelle 1 zusammengefaßt.

Tabelle 1, Teil 1:

| Verbindung der Formel I $R^1$ | $R^2$ | $R^5$ | $R^6$ | $R^7$ | $R^{8a}$ | $R^8$ | Substanz No. (Beispiel) |
|---|---|---|---|---|---|---|---|
| Adriamycin | | | | | | | |
| H | OH | OH | $CH_2CH_3$ | H | - | H | (1) |
| H | OH | OH | $CH_2CH_3$ | H | - | H | (2) |
| OH | OH | OH | $CH_2CH_3$ | H | - | H | (3) |
| OH | OH | OH | $CH_2CH_3$ | H | - | H | (4) |
| OH | OH | $COOCH_3$ | $CH_2CH_3$ | H | - | H | (5) |
| H | OH | OH | $CH_2CH_3$ | H | - | CHO | (6) |
| H | OH | OH | $CH_2CH_3$ | H | - | $CH_2CN$ | (7) |
| OH | OH | OH | $CH_2CH_3$ | H | - | $CH_2CN$ | (8) |
| H | OH | OH | $CH_2CH_3$ | H | - | Benzyl | (9) |
| OH | OH | OH | $CH_2CH_3$ | H | - | Benzyl | (10) |
| H | OH | OH | $CH_2CH_3$ | H | - | $CH_2CH_3$ | (11) |
| H | OH | OH | $CH_2CH_3$ | H | - | Propyl | (12) |
| H | OH | OH | $CH_2CH_3$ | H | - | Butyl | (13) |
| OH | OH | $COOCH_3$ | $CH_2CH_3$ | H | - | $COCH_3$ | (14) |
| H | OH | II | $CH_2CH_3$ | H | H | H | (15) |
| H | OH | III | $CH_2CH_3$ | H | - | H | (17) |

a) für die genannten Verbindungen gilt $R^3=R^4=OH$

Tabelle 1, Teil 2:

| $R^1$ | $R^2$ | $R^5$ | $R^6$ | $R^{8a}$ | $R^8$ | Substanz No. (Beispiel) |
|---|---|---|---|---|---|---|
| H | OH | OH | $CH_2CH_3$ | - | Pentyl | (18) |
| H | OH | OH | $CH_2CH_3$ | - | Hexyl | (19) |
| H | OH | OH | $CH_2CH_3$ | - | Heptyl | (20) |
| H | OH | OH | $CH_2CH_3$ | - | Octyl | (21) |
| H | OH | OH | $CH_2CH_3$ | - | 4-Chlorbenzyl | (22) |
| H | OH | OH | $CH_2CH_3$ | - | 4-Brombenzyl | (23) |
| H | OH | OH | $CH_2CH_3$ | - | 4-Nitrobenzyl | (24) |
| H | OH | OH | $CH_2CH_3$ | - | 4-Cyanobenzyl | (25) |
| H | OH | OH | $CH_2CH_3$ | - | Cyclohexylmethyl | (26) |
| OH | OH | OH | $CH_2CH_3$ | - | Pentyl | (27) |
| H | OH | II | $CH_2CH_3$ | Benzyl | Benzyl | (28) |
| H | OH | II | $CH_2CH_3$ | Pentyl | Pentyl | (29) |
| H | OH | II | $CH_2CH_3$ | Cyanomethyl | Cyanomethyl | (30) |
| H | $OCH_3$ | H | $COCH_3$ | - | H | (31) |
| H | $OCH_3$ | H | $CHOHCH_3$ | - | H | (32) |

a) für die genannten Verbindungen gilt $R^3$=$R^4$=OH und $R^7$=H

Tabelle 1, Teil 3:

| Substanz No. (Beispiel) | $R_F$-Werte [a)] Laufmittel 1 | 2 | 3 | Dauerinkubation $IC_{50}$ (µg/ml) | 1 h-Inkubation $IC_{50}$ (µg/ml) |
|---|---|---|---|---|---|
| | | | | 0.02 | 0.04 |
| (1) | 0.86 | 0.60 | 0.29 | 0.02 | 0.038 |
| (2) | 0.86 | 0.60 | | 0.02 | 0.026 |
| (3) | 0.78 | 0.48 | | 0.11 | 0.13 |
| (4) | 0.78 | 0.48 | 0.1 | 0.11 | 0.13 |
| (5) | 0.87 | 0.20 | 0.13 | 0.28 | > 1 |
| (6) | 0.88 | 0.65 | 0.66 | 0.044 | 0.15 |
| (7) | 0.82 | 0.68 | 0.53 | 0.013 | 0.021 |
| (8) | 0.83 | 0.69 | 0.48 | 0.042 | 0.2 |
| (9) | 0.78 | 0.50 | 0.46 | 0.028 | 0.32 |
| (10) | 0.78 | 0.49 | 0.45 | | |
| (11) | 0.53 | 0.30 | 0.09 | 0.007 | 0.08 |
| (12) | 0.65 | 0.38 | 0.18 | 0.026 | 0.1 |
| (13) | 0.75 | 0.45 | 0.29 | 0.018 | 0.125 |
| (14) | | | | 0.5 | > 1 |
| (15) | 0.43 | 0.20 | 0.13 | > 1 | > 1 |
| (17) | 0.38[b)] | | | 0.0024 | 0.06 |

Tabelle 1, Teil 4:

| Substanz No. (Beispiel) | $R_F$-Werte Laufmittel 1 | 2 | 3 | Dauerinkubation $IC_{50}$ (µg/ml) | 1h-Inkubation $IC_{50}$ (µg/ml) |
|---|---|---|---|---|---|
| (18) | 0.78 | 0.52 | 0.30 | 0.075 | 0.48 |
| (19) | 0.81 | 0.59 | 0.32 | 0.13 | 1.2 |
| (20) | 0.68 | 0.48 | 0.31 | 0.38 | 1.9 |
| (21) | 0.65 | 0.49 | 0.28 | 0.46 | 2.8 |
| (22) | 0.67 | 0.41 | 0.61 | 0.026 | 0.95 |
| (23) | 0.74 | 0.47 | 0.70 | 0.08 | > 1 |
| (24) | 0.69 | 0.52 | 0.83 | 0.16 | 2.5 |
| (25) | 0.66 | 0.49 | 0.81 | 0.11 | > 1 |
| (26) | 0.73 | 0.48 | 0.39 | | |
| (27) | 0.68 | 0.45 | 0.20 | 0.06 | > 1 |
| (28) | 0.60 | 0.40 | 0.40 | 0.0065 | 0.12 |
| (29) | 0.57 | 0.36 | 0.08 | 0.1 | 0.6 |
| (30) | 0.79 | 0.68 | 0.51 | | |
| (31) | 0.50 | 0.29 | 0.01 | | |
| (32) | 0.35 | 0.16 | 0.009 | 0.5 | 1.3 |

Anmerkungen zur Tabelle 1

a) Laufmittel 1 : Chloroform / Methanol / Essigsäure / Wasser / Triäthylamin (80/20/10/4/0,2)

Laufmittel 2 : Laufmittel 1 / Dichlormethan (2/1)

Laufmittel 3 : Dichlormethan / Methanol (9/1)

b) Chloroform / Methanol / Essigsäure / Wasser (8/1/1/0,2)

Bei den Zahlenangaben handelt es sich um Volumenverhältnisse.

Zur Erläuterung des erfindungsgemäßen Herstellungsverfahrens werden nachfolgend Beispiele 1 bis 32 aufgeführt, in denen bevorzugte erfindungsgemäße Verbindungen nach dem beanspruchten Verfahren hergestellt wurden. Die Struktur der hergestellten Verbindung wurde mittels $^{1}H$- und $^{13}C$-NMR sowie MS-Spektroskopie ermittelt. Der Verlauf der Reaktionen sowie die resultierenden Verbindungen wurden dünnschichtchromatographisch oder mit der HPLC-Technik untersucht.

Dünnschichtchromatographie erfolgte, sofern nicht anders vermerkt, auf Kieselgel - Fertigplatten in Laufmittel 1: Methanol (20 Teile)/Chloroform (80 Teile)/Essigsäure (10 Teile)/Wasser (4 Teile)/Triäthylamin (0,2 Teile). Säulenchromatographie erfolgte, sofern nicht anders vermerkt, über Kieselgel 60 vom Durchmesser 20 - 45 um oder 0,063 - 0,200 mm. Vorstehende Teile sind Volumenteile. Die Ausbeuten sind nicht optimiert.

Beispiel 1:

7-O-(3'-N-Methyl-α-L-daunosaminyl)-β-rhodomycinon (Verbindung 1)

(Verbindung der Formel I mit $R^1 = H$, $R^2 = R^3 = R^4 = R^5 = OH$, $R^6 = CH_2CH_3$ und $R^7 = R^8 = H$)

Eine Lösung von 500 mg = 0,92 mmol 7-O-α-L-Rhodosaminylβ-rhodomycinon, das nach bekannten Verfahren, z.B. Yoshimoto et al. 1984, J. Antibiot. (1984) 37, 920, erhalten worden war, in einer Mischung aus Chloroform (1200 ml) und Methanol (50 ml) wurde auf 8 Petrischalen vom Durchmesser 19 cm verteilt und auf einer reflektierenden Unterlage unter leichtem Rühren aus ca. 25 cm Abstand mit zwei 500 Watt-Strahlern 5 Stunden lang bestrahlt, wobei die Reaktion dünnschichtchromatographisch verfolgt wurde.

Dann wurden die Lösungen vereint und das Lösungsmittel am Rotationsverdampfer abgezogen. Der Rückstand wurde in möglichst wenig Methanol gelöst, mit Wasser versetzt, mit 1% Salzsäure auf pH 4 eingestellt und mehrfach mit Chloroform extrahiert, wobei das gewünschte Produkt in der wässrigen Phase verblieb. Diese"Chloroform-Phase I" wurde eingeengt und wie im Beispiel 6 beschrieben weiterverarbeitet. Die wässrige Phase wurde mit einer gesättigten wässrigen Lösung von Natriumhydrogencarbonat auf pH 7-8 eingestellt und das gewünschte Produkt mit Chloroform extrahiert.
Ausbeute: 264 mg (0,50 mmol) = 54%.

Beispiel 2:

7-O-(3'-N-Methyl-α-L-daunosaminyl)-β-rhodomycinon-hydrochlorid (Verbindung 2)

(HCl-Addukt einer Verbindung der Formel I mit $R^1 = H$, $R^2 = R^3 = R^4 = R^5 = OH$, $R^6 = CH_2CH_3$ und $R^7 = R^8 = H$)

500 mg 7-O-α-L-Rhodosaminyl-β-rhodomycinon wurden in Analogie zu Beispiel 1 demethyliert und aufgearbeitet. Die mit 1% Salzsäure auf pH4 eingestellte wässrige Lösung wurde nach mehrfacher Extraktion mit Chloroform lyophilisiert.
Ausbeute: 282 mg (0,50 mmol) = 54%.

Beispiel 3:

7-O-(3'-N-Methyl-α-L-daunosaminyl)-β-isorhodomycinon (Verbindung 3)

(Verbindung der Formel I mit $R^1 = R^2 = R^3 = R^4 = R^5 = OH$, $R^6 = CH_2CH_3$ und $R^7 = R^8 = H$)

280 mg (0,50 mmol) 7-O-α-L-Rhodosaminyl-β-isorhodomycinon, das durch milde Säurehydrolyse des literaturbekannten beta-Isorhodomycin-II (Brockmann et al., Tetrahedron Letters 1969, 415) in Analogie zur Darstellung von 7-O-α-L-Rhodosaminyl-β-rhodomycinon aus β-Rhodomycin-II erhalten worden war, wurden in 60 ml Methanol gelöst, mit 1000 ml Chloroform versetzt und 90 Minuten lang wie in Beispiel 1 bestrahlt.Dann wurde das Lösungsmittel am Rotationsverdampfer abgezogen, der Rückstand in wenig Methanol aufgenommen, mit 1% Salzsäure auf pH 1 eingestellt, mit Wasser auf ca. 400 ml verdünnt und mehrmals mit Chloroform extrahiert, wobei das gewünschte Produkt in der wässrigen Phase verblieb. Schließlich wurde mit einer gesättigten wässrigen Lösung von Natriumhydrogencarbonat auf pH 7 eingestellt und das gewünschte Produkt mit Chloroform extrahiert.
Ausbeute: 234 mg (0,43 mmol) = 86%

Gegebenenfalls kann das Produkt im Laufmittel Dichlormethan/Methanol (4/1) über eine Kieselgel 60-Säule chromatographisch gereinigt werden.

Beispiel 4:

7-O-(3'-N-Methyl-α-L-daunosaminyl)-β-isorhodomycinonhydrochlorid (Verbindung 4)

(HCl-Addukt einer Verbindung der Formel I mit $R^1 = R^2 = R^3 = R^4 = R^5 = OH$, $R^6 = CH_2CH_3$ und $R^7 = R^8 = H$)

50 mg (0,09 mmol) 7-O-α-L-Rhodosaminyl-β-isorhodomycinon wurden in Analogie zu Beispiel 3 demethyliert und aufgearbeitet. Die mit 1% Salzsäure angesäuerte wässrige Lösung wurde nach mehrfacher Extraktion mit Chloroform lyophilisiert.
Ausbeute: 41 mg (0,07 mmol) = 78%

Beispiel 5:

7-O-(3'-N-Methyl-α-L-daunosaminyl)-ε-isorhodomycinon (Verbindung 5)

(Verbindung der Formel I mit $R^1 = R^2 = R^3 = R^4 = OH$, $R^5 = COOCH_3$, $R^6 = CH_2CH_3$ und $R^7 = R^8 = H$)

62 mg (0,10 mmol) 7-O-α-L-Rhodosaminyl-ε-isorhodomycinon wurden in Analogie zu Beispiel 1 demethyliert und die Umsetzung dünnschichtchromatographisch verfolgt. Nach Verbrauch der Ausgangsverbindung wurde das Lösungsmittel am Rotationsverdampfer abgezogen und das Reaktionsprodukt wiederholt säulenchromatographisch (10 g Kieseigel 60 für HPLC, 25-40 µm, Merck; Laufmittel: Dichlormethan/Methanol/Wasser (80/8/1) getrennt. Zur Entfernung von Kieselgel-Resten wurde das gereinigte Festprodukt mehrmals mit Chloroform extrahiert und aus den vereinigten Chloroformphasen zurückgewonnen.
Ausbeute: 27 mg (nicht optimiert).
Der Molpeak des Fab-Massenspektrums ($M + H^+ = 588$) steht im Einklang mit der berechneten Molmasse von 587,6 ($C_{29}H_{33}NO_{12}$).

Beispiel 6:

7-O-(3'-N-Formyl-3'-N-methyl-α-L-daunosaminyl)-β-rhodomycinon (Verbindung 6)

(Verbindung der Formel I mit $R^1 = H$, $R^2 = R^3 = R^4 = R^5 = OH$, $R^6 = CH_2CH_3$, $R^7 = H$ und $R^8 = CHO$)

Verschiedene in Analogie zu Beispiel I gewonnene Produktgemische der "Chloroform-Phase I" wurden vereint (390 mg) und über eine Kieselgelsäule (Durchmesser 3,5 cm, Füllhöhe 10 cm; Laufmittel: Dichlormethan/Methanol (19/1)) unter geringfügigem Druck (Zuhilfenahme von Druckluft) getrennt. Die Fraktionen mit Reinsubstanz von $R_F$ 0,29 in Dichlormethan/Methanol (19/1) wurden gesammelt.
Ausbeute: 80 mg
Der Molpeak des Fab-Massenspektrums ($M + H^+ = 588$) steht im Einklang mit der berechneten Molmasse von 557,5 ($C_{28}H_{31}NO_{11}$).

Beispiel 7:

7-O-(3'-N-Cyanomethyl-3'-N-methyl-α-L-daunosaminyl)-β-rhodomycinon (Verbindung 7)

(Verbindung der Formel I mit $R^1 = H$, $R^2 = R^3 = R^4 = R^5 = OH$, $R^6 = CH_2CH_3$, $R^7 = H$ und $R^8 = CH_2CN$)

Zu einer Lösung von 7-O-(3'-N-Methyl-α-L-daunosaminyl)-β-rhodomycinon (200 mg = 0,38 mmol) und Triäthylamin (1,14 mmol) in 10 ml trockenem Dimethylformamid wurden 630 mg (3,8 mmol = 10 äquiv.) Jodacetonitril zugesetzt und über Nacht bei Raumtemperatur gerührt. Dann wurde am Rotationsverdampfer eingeengt. Der Rückstand wurde in Chloroform aufgenommen, die Lösung dreimal mit Wasser extrahiert, mit Natriumsulfat getrocknet, filtriert und das Lösungsmittel entfernt. Der Rückstand wurde über eine Kieselgel-Säule mit Dichlormethan/Methanol (95/5), getrennt ($R_F$ 0,28).
Ausbeute: 105 mg (0,18 mmol) = 47%.

Beispiel 8:

7-O-(3'-N-Cyanomethyl-3'-N-methyl-α-L-daunosaminyl)-β-isorhodomycinon (Verbindung 8)

(Verbindung der Formel I mit $R^1 = R^2 = R^3 = R^4 = R^5 = OH$ $R^6 = CH_2CH_3$, $R^7 = H$ und $R^8 = CH_2CN$)

30 mg 7-O-(3'-N-Methyl-α-L-daunosaminyl)-β-isorhodomycinon wurden in Analogie zu Beispiel 7 mit Jodacetonitril umgesetzt und aufgearbeitet und über eine Kieselgel 60-Säule mit Laufmittel Dichlormethan/Methanol (99/1) gereinigt.
Ausbeute: 8 mg

Beispiel 9:

7-O-(3'-N-Benzyl-3'-N-methyl-α-L-daunosaminyl)-β-rhodomycinon (Verbindung 9)

(Verbindung der Formel I mit $R^1 = H$, $R^2 = R^3 = R^4 = R^5 = OH$, $R^6 = CH_2CH_3$, $R^7 = H$ und $R^8$ = Benzyl)

Eine Lösung von 52 mg (0,09 mmol) 7-O-(3'-N-Methyl-α-L-daunosaminyl)-β-rhodomycinon und 190 µl (1,8 mmol = 20 äquiv.) Benzaldehyd in 3 ml Acetonitril/Wasser (3/1) wurde mit 6 µl (0,1 mmol) Eisessig versetzt. Nach 4 h wurde eine Lösung von 20 mg (0,6 mmol) Natriumcyanoborhydrid in 900 µl Acetonitril/Wasser (3/1) zugesetzt und im Dunkeln drei Tage weitergerührt. Dann wurde mit 15 ml Wasser versetzt und die Lösung dreimal mit jeweils 20 ml Chloroform ausgeschüttelt. Die vereinigten Chloroform-Phasen wurden nacheinander mit Wasser und gesättigter Natriumhydrogencarbonat-Lösung rückextrahiert, das Lösungsmittel abgezogen, und das erhaltene Produkt über eine Kieselgel 60-Säule mit Laufmittel Dichlormethan/Methanol (99/1) chromatographiert.
Ausbeute: 30 mg (0,05 mmol) = 56%

Beispiel 10:

7-O-(3'-N-Benzyl-3'-N-methyl-α-L-daunosaminyl)-β-isorhodomycinon (Verbindung 10)

(Verbindung der Formel I mit $R^1 = R^2 = R^3 = R^4 = R^5 = OH$, $R^6 = CH_2CH_3$, $R^7 = H$ und $R^8$ = Benzyl)

30 mg 7-O-(3'-N-Methyl-α-L-daunosaminyl)-β-isorhodomycinon wurden in Analogie zu Beispiel 9 mit Benzaldehyd umgesetzt und aufgearbeitet und über eine Kieselgel-Säule mit Laufmittel Dichlormethan/Methanol (99/1) gereinigt.
Ausbeute: 5 mg

Beispiel 11:

7-O-(3'-N-Äthyl-3'-N-methyl-α-L-daunosaminyl)-β-rhodomycinon (Verbindung 11)

(Verbindung der Formel I mit $R^1 = H$, $R^2 = R^3 = R^4 = R^5 = OH$, $R^6 = CH_2CH_3$, $R^7 = H$ und $R^8 = CH_2CH_3$)

52 mg 7-O-(3'-N-Methyl-α-L-daunosaminyl)-β-rhodomycinon wurden in Analogie zu Beispiel 9 mit Acetaldehyd umgesetzt und aufgearbeitet und über eine Kieselgel-Säule mit Laufmittel-Gradienten Dichlormethan/Methanol (99/1) bis (8/2) gereinigt.
Ausbeute: 14 mg

Beispiel 12:

7-O-(3'-N-Methyl-3'-N-propyl-α-L-daunosaminyl)-β-rhodomycinon (Verbindung 12)

(Verbindung der Formel I mit $R^1 = H$, $R^2 = R^3 = R^4 = R^5 = OH$, $R^6 = CH_2CH_3$, $R^7 = H$ und $R^8$ = Propyl)

52 mg 7-O-(3'-N-Methyl-α-L-daunosaminyl)-β-rhodomycinon wurden in Analogie zu Beispiel 9 mit Propionaldehyd umgesetzt und aufgearbeitet und über eine Kieselgel-Säule mit Laufmittel-Gradienten Dichlormethan/Methanol (99/1) bis (9/1) gereinigt.

Ausbeute: 18 mg

Beispiel 13:

7-O-(3'-N-Butyl-3'-N-methyl-α-L-daunosaminyl)-β-rhodomycinon (Verbindung 13)

(Verbindung der Formel I mit $R^1 = H$, $R^2 = R^3 = R^4 = R^5 = OH$, $R^6 = CH_2CH_3$, $R^7 = H$ und $R^8$ = Butyl)

52 mg 7-O-(3'-N-Methyl-α-L-daunosaminyl)-β-rhodomycinon wurden in Analogie zu Beispiel 9 mit Butyraldehyd umgesetzt und aufgearbeitet und über eine Kieselgel-Säule mit Laufmittel-Gradienten Dichlormethan/Methanol (99/1) bis (9/1) gereinigt.
Ausbeute: 20 mg

Beispiel 14:

7-O-(3'-N-Acetyl-3'-N-methyl-α-L-daunosaminyl)-ε-isorhodomycinon (Verbindung 14)

(Verbindung der Formel I mit $R^1 = R^2 = R^3 = R^4 = OH$, $R^5 = COOCH_3$, $R^6 = CH_2CH_3$, $R^7 = H$ und $R^8 = COCH_3$) $R^6 = CH_2CH_3$, $R^7 = H$ und $R^8 = COCH_3$)

15 mg 7-O-(3'-N-Methyl-α-L-daunosaminyl)-ε-isorhodomycinon wurden in 3 ml Methanol mit 100 µl Acetanhydrid N-acetyliert. Dann wurde am Rotationsverdampfer eingeengt, mit gesättigter Natriumhydrogencarbonatlösung aufgenommen und das Reaktionsprodukt mit Chloroform extrahiert.
Ausbeute: 11 mg

Beispiel 15:

7,10-O-Bis-(3'-N-methyl-α-L-daunosaminyl)-β-rhodomycinon (Verbindung 15)

(Verbindung der Formel I mit $R^1 = H$, $R^2 = R^3 = R^4 = OH$, $R^5$ = Struktur der Formel II mit $R^{8a} = H$ $R^6 = CH_2CH_3$ und $R^7 = R^8 = H$)

120 mg 7,10-O-Bis-α-L-rholosaminyl-β-rhodomycinon (Australische Patentanmeldung 84/30823) wurden in Analogie zu Beispiel 1 drei Stunden lang bestrahlt. Dann wurde das Lösungsmittel am Rotationsverdampfer abgezogen. Der Rückstand wurde in wenig Methanol aufgenommen, mit Wasser versetzt und nacheinander bei pH 2,4,6 und 8 mit Chloroform extrahiert. Das Produkt der bei pH 6 und 8 gewonnenen Chloroform-Extrakte wurde über eine KieselgelSäule mit Laufmittel 1, welches im Verhältnis 1:1 mit Dichlormethan versetzt war, chromatographiert und nach erneutem Ausschütteln mit wässrigem Natriumhydrogencarbonat/Chloroform in reiner Form isoliert.
Ausbeute: 66 mg

Beispiel 16:

Monodemethyliertes Cytorhodin T (Verbindung 17)

(Verbindung der Formel I mit $R^1 = H$, $R^2 = R^3 = R^4 = OH$, $R^5$ = Struktur der Formel III, $R^6 = CH_2CH_3$ und $R^7$ und $R^8$ = H)

Cytorhodin T ( gemäß AU 84/30823) wurde in Analogie zu Beispiel 1 photolysiert. Alternativ wurden 50 mg (0,053 mmol) Cytorhodin T in 5 ml Chloroform aufgenommen und bei Raumtemperatur unter Tageslichteinfluß stehengelassen. Die Reaktion wurde dünnschichtchromatographisch verfolgt. Nach 7 Tagen wurde vom Lösungsmittel befreit und der Rückstand über präparative Dünnschichtchromatographie in dem System: Chloroform/Methanol/ 99%Essigsäure/Wasser = 8/1/1/0,2) gereinigt.
Ausbeute: 16 mg

Beispiel 17:

7-O-(3'-N-Methyl-3'-N-pentyl-α-L-daunosaminyl)-β-rhodomycinon (Verbindung 18)

(Verbindung der Formel I mit $R^1 = H$, $R^2 = R^3 = R^4 = R^5 = OH$, $R^6 = CH_2CH_3$, $R^7 = H$ und $R^8$ = Pentyl)

52 mg 7-O-(3'-N-Methyl-α-L-daunosaminyl)-β-rhodomycinon wurden in Analogie zu Beispiel 9 mit Pentanal umgesetzt und aufgearbeitet und über eine Kieselgel-Säule im Laufmittel Dichlormethan/Methanol (95/5) gereinigt.
Ausbeute: 25 mg (48 %)

Beispiel 18:

7-O-(3'-N-Hexyl-3'-N-methyl-α-L-daunosaminyl)-β-rhodomycinon (Verbindung 19)

(Verbindung der Formel I mit $R^1 = H$, $R^2 = R^3 = R^4 = R^5 = OH$, $R^6 = CH_2CH_3$, $R^7 = H$ und $R^8$ = Hexyl)

52 mg 7-O-(3'-N-Methyl-α-L-daunosaminyl)-β-rhodomycinon wurden in Analogie zu Beispiel 9 mit Hexanal umgesetzt und aufgearbeitet und über eine Kieselgel-Säule im Laufmittel Dichlormethan/Methanol (9/1) gereinigt.
Ausbeute: 25 mg (48 %)

Beispiel 19:

7-O-(3'-N-Heptyl-3'-N-methyl-α-L-daunosaminyl)-β-rhodomycinon (Verbindung 20)

(Verbindung der Formel I mit $R^1 = H$, $R^2 = R^3\text{-}R^4\text{-}R^5 = OH$, $R^6 = CH_2CH_3$, $R^7 = H$ und $R^8$ = Heptyl)

58 mg 7-O-(3'-N-Methyl-α-L-daunosaminyl)-β-rhodomycinon wurden in Analogie zu Beispiel 9 mit Heptanal umgesetzt und aufgearbeitet und über eine Kieselgel-Säule im Laufmittel Dichlormethan/Methanol (9/1) gereinigt.
Ausbeute: 34 mg (59 %)

Beispiel 20:

7-O-(3'-N-Octyl-3'-N-methyl-α-L-daunosaminyl)-β-rhodomycinon (Verbindung 21)

(Verbindung der Formel I mit $R^1 = H$, $R^2 = R^3 = R^4 = R^5 = OH$, $R^6 = CH_2CH_3$, $R^7 = H$ und $R^8$ = Octyl)

58 mg 7-O-(3'-N-Methyl-α-L-daunosaminyl)-β-rhodomycinon wurden in Analogie zu Beispiel 9 mit Octanal umgesetzt und aufgearbeitet und über eine Kieselgel-Säule im Laufmittel Dichlormethan/Methanol (9/1) gereinigt.
Ausbeute: 40 mg (69 %)

Beispiel 21:

7-O-(3'-N-(4-Chlorbenzyl)-3'-N-methyl-α-L-daunosaminyl)-β-rhodomycinon (Verbindung 22)

(Verbindung der Formel I mit $R^1 = H$, $R^2 = R^3 = R^4 = R^5 = OH$, $R^6 = CH_2CH_3$, $R^7 = H$ und $R^8$ = 4-Chlorbenzyl)

52 mg 7-O-(3'-N-Methyl-α-L-daunosaminyl)-β-rhodomycinon wurden in Analogie zu Beispiel 9 mit 4-Chlorbenzaldehyd umgesetzt und aufgearbeitet und über eine Kieselgel-Säule im Laufmittel Dichlormethan/Methanol (975/25) gereinigt.
Ausbeute: 31 mg (60 %)

Beispiel 22:

7-O-(3'-N-(4-Brombenzyl)-3'-N-methyl-α-L-daunosaminyl)-β-rhodomycinon (Verbindung 23)

(Verbindung der Formel I mit $R^1 = H$, $R^2 = R^3 = R^4 = R^5 = OH$, $R^6 = CH_2CH_3$, $R^7 = H$ und $R^8$ = 4-Brombenzyl)

52 mg 7-O-(3'-N-Methyl-α-L-daunosaminyl)-β-rhodomycinon wurden in Analogie zu Beispiel 9 mit 4-Brombenzaldehyd umgesetzt und aufgearbeitet und über eine Kieselgel-Säule im Laufmittel Dichlormethan/Methanol (95/5) gereinigt.
Ausbeute: 30 mg (58 %)

Beispiel 23:

7-O-(3'-N-(4-Nitrobenzyl)-3'-N-methyl-α-L-daunosaminyl)-β-rhodomycinon (Verbindung 24)

(Verbindung der Formel I mit $R^1 = H$, $R^2 = R^3 = R^4 = R^5 = OH$, $R^6 = CH_2CH_3$, $R^7 = H$ und $R^8$ = 4-Nitrobenzyl)

58 mg 7-O-(3'-N-Methyl-α-L-daunosaminyl)-β-rhodomycinon wurden in Analogie zu Beispiel 9 mit 4-Nitrobenzaldehyd umgesetzt und aufgearbeitet, dann über eine Kieselgel-Säule im Laufmittel Toluol/Äthanol (965/35) gereinigt.
Ausbeute: 30 mg (52 %)

Beispiel 24:

7-O-(3'-N-(4-Cyanobenzyl)-3'-N-methyl-α-L-daunosaminyl)-β-rhodomycinon (Verbindung 25)

(Verbindung der Formel I mit $R^1 = H$, $R^2 = R^3 = R^4 = R^5 = OH$, $R^6 = CH_2CH_3$, $R^7 = H$ und $R^8$ = 4-Cyanobenzyl)

58 mg 7-O-(3'-N-Methyl-α-L-daunosaminyl)-β-rhodomycinon wurden in Analogie zu Beispiel 9 mit 4-Cyanobenzaldehyd umgesetzt und aufgearbeitet und über eine Kieselgel-Säule im Laufmittel Dichlormethan/Methanol (975/25) gereinigt.
Ausbeute: 49 mg (84 %)

Beispiel 25:

7-O-(3'-N-Cyclohexylmethyl-3'-N-methyl-α-L-daunosaminyl)-β-rhodomycinon (Verbindung 26)

(Verbindung der Formel I mit $R^1 = H$, $R^2 = R^3 = R^4 = R^5 = OH$, $R^6 = CH_2CH_3$, $R^7 = H$ und $R^8$ = Cyclohexylmethyl)

20 mg 7-O-(3'-N-Methyl-α-L-daunosaminyl)-β-rhodomycinon wurden in Gegenwart von 4.6 µl Essigsäure mit 170 mg Cyclohexanaldehyd (184 µl ≙ 40 equiv.) in einem Lösungsmittelgemisch aus Acetonitril/Wasser (4/1) 10 min. bei Raumtemperatur gerührt. Dann wurden erneut 4.6 µl Essigsäure zugesetzt und die Reaktion bis zum Verschwinden der Ausgangsverbindung weitergerührt. Dann wurde die Reaktionslösung in 0.1 N Salzsäure gegossen, zweimal mit n-Hexan ausgewaschen, die wäßrige Phase mit festem Natriumhydrogencarbonat auf pH 11 gebracht, das Reaktionsprodukt mit Chloroform extrahiert und über eine Kieselgel-Säule in einem Laufmittel, bestehend aus Laufmittel 1 und Dichlormethan im Verhältnis 1/2, gereinigt. Restliche Essigsäure wurde durch erneutes Ausschütteln mit Chloroform/Natriumhydrogencarbonatlösung entfernt.
Ausbeute: 11 mg (47 %)

Beispiel 26:

7-O-(3'-N-Methyl-3'-N-pentyl-α-L-daunosaminyl)-β-isorhodomycinon (Verbindung 27)

(Verbindung der Formel I mit $R^1 = R^2 = R^3 = R^4 = R^5 = OH$, $R^6 = CH_2CH_3$, $R^7 = H$ und $R^8$ = Pentyl)

20 mg 7-O-(3'-N-Methyl-α-L-daunosaminyl)-β-isorhodomycinon wurden in Analogie zu Beispiel, 9 mit Pentanal umgesetzt und aufgearbeitet und über eine Kieselgel-Säule im Laufmittel Dichlormethan/Methanol (9/1) gereinigt.
Ausbeute: 6 mg (30 %)

Beispiel 27:

7,10-O-Bis-(3'-N-benzyl-3'-N-methyl-α-L-daunosaminyl)-β-rhodomycinon (Verbindung 28)

(Verbindung der Formel I mit $R^1 = H$, $R^2 = R^3 = R^4 = OH$, $R^5$ = Struktur der Formel II mit $R^{8a}$ = Benzyl, $R^6 = CH_2CH_3$, $R^7 = H$ und $R^8$ = Benzyl)

122 mg (0.18 mmol) 7,10-O-Bis-(3'-N-methyl-α-L-daunosaminyl)-β-rhodomycinon (Verbindung 15) wurden mit 1.01 g (9.5 mmol) Benzaldehyd in Gegenwart von 22 mg Essigsäure und 288 mg Natriumcyanoborhydrid in Acetonitril/Wasser (3/1) (8 ml) über Nacht gerührt. Dann wurde mit 40 ml Wasser versetzt, mit Salzsäure auf pH 1 gestellt und mit Chloroform ausgewaschen. Die wäßrige Phase wurde mit gesättigter Natriumhydrogencarbonat-Lösung auf pH 8 gestellt und mit Chloroform extrahiert. Das extrahierte Produktgemisch (110 mg) wurde über eine 20 g Kieselgel-Säule mit Laufmittel 1/Dichlormethan (1/1) aufgetrennt (Ausbeute: 24 mg) und über eine 5 g Kieselgel-Säule mit Laufmittel Dichlormethan/Methanol (20/1) nachgereinigt.
Ausbeute: 13 mg

Beispiel 28:

7,10-O-Bis-(3'-N-methyl-3'-N-pentyl-α-L-daunosaminyl)-β-rhodomycinon (Verbindung 29)

(Verbindung der Formel I mit $R^1 = H$, $R^2 = R^3 = R^4 = OH$, $R^5$ = Struktur der Formel II ,mit $R^{8a}$ = Pentyl, $R^6 = CH_2CH_3$, $R^7 = H$ und $R^8$ = Pentyl)

61 mg (0.09 mmol) 7,10-O-Bis-(3'-N-methyl-α-L-daunosaminyl)-β-rhodomycinon (Verbindung 15) wurden mit 288 mg (3.34 mmol) Valeraldehyd in Gegenwart von 11 mg Essigsäure und 144 mg Natriumcyanoborhydrid in Acetonitril/Wasser (3/1) (3 ml) in Analogie zu Beispiel 29 umgesetzt. Nach 1 h wurde mit 50 ml Wasser versetzt und mehrmals mit Chloroform extrahiert. Das extrahierte Produktgemisch wurde über eine 10 g Kieselgel-Säule mit Laufmittel 2 aufgetrennt und das gewünschte Produkt mit Dichlormethan gegen eine wäßrige Lösung von Natriumhydrogencarbonat ausgeschüttelt.
Ausbeute: 27 mg (37 %)

Beispiel 29:

7,10-O-Bis-(3'-N-cyanomethyl-3'-N-methyl-α-L-daunosaminyl)-β-rhodomycinon (Verbindung 30)

(Verbindung der Formel I mit $R^1 = H$, $R^2 = R^3 = R^4 = OH$, $R^5$ = Struktur der Formel II mit $R^{8a}$ = Cyanomethyl, $R^6 = CH_2CH_3$, $R^7 = H$ und $R^8$ = Cyanomethyl)

84 mg (0.125 mmol) 7,10-O-Bis-(3'-N-methyl-α-L-daunosaminyl)-β-rhodomycinon (Verbindung 15) und 175 µl (2.5 mmol) Jodacetonitril wurden in trockenem Dimethylformamid (5 ml) in Gegenwart von 400 µl Triethylamin in Analogie zu Beispiel 7 umgesetzt und aufgearbeitet. Das Produktgemisch wurde über eine 30 g Kieselgel-Säule anfänglich mit Chloroform und nachfolgend mit einer Mischung aus Chloroform/Methanol (9/1) eluiert.
Ausbeute: 49 mg (52 %)

Beispiel 30:

3'-N-Methyl-daunomycin (Verbindung 31)

(Verbindung 'der Formel I mit $R^1 = H$, $R^2 = OCH_3$, $R^3 = R^4 = OH$, $R^5 = H$, $R^6 = COCH_3$ und $R^7 = R^8 = H$)

95 mg (0.17 mmol) N,N-Dimethyldaunomycin (Tong et al., J. Med. Chem. (1979), 22, 912) wurden in Analogie zu Beispiel 1 demethyliert und aufgearbeitet.
Ausbeute: 29 mg (31 %)

Beispiel 31:

3'-N-Methyl-13-dihydrodaunorubicin (Verbindung 32)

(Verbindung der Formel I mit $R^1 = H$, $R^2 = OCH_3$, $R^3 = R^4 = OH$, $R^5 = H$, $R^6 = CHOHCH_3$ und $R^7 = R^8 = H$)

50 mg (0.09 mmol) N,N-Dimethyl-13-dihydrodaunorubicin (Tong et al., J. Med. Chem. (1979), 22, 912) wurden in Analogie zu Beispiel 1 demethyliert und aufgearbeitet.
Ausbeute: 6.5 mg (13 %)
In der nachfolgenden Tabelle 2 sind die nmr - Daten der vorstehend beschriebenen neuen Verbindungen 1 bis 17 zusammengestellt, in der Tabelle 3 sind die nmr - Daten der vorstehend beschriebenen neuen Verbindungen 18 - 32 zusammengestellt.

Tabelle 2

400 MHz $^{1}$H-nmr-Daten verschiedener Verbindungen der Formel I

die Substanz-Nummern der ersten Zeile entsprechen den jeweiligen Beispielsnrn.;
die Indizes a) bis u) dienen der näheren Charakterisierung;

Abkürzungen s: Singulett
d: Dublett
t: Triplett
q: Quartett
dd: Dublett eines Dubletts
bs: breites Singulett

a) Spektrum gemessen in $CDCl_3/D_6$-DMSO gegen TMS
b) 300 MHz-Spektrum
c) Spektrum gemessen in $CDCl_3$ gegen TMS
d) für beide Rotamere werden isolierte Signale beobachtet
e) CHO: 8.12 s; 8.01 s
f) $NCH_2CN$: 3.59 dd
g) $NCH_2Ph$: ß:3.62 d; α:3.40 d
h) Wert in $CD_3OD$
i) nicht eindeutig identifizierbar
k) Zuordnung der OH-Gruppen nicht eindeutig
l) Daten von einem anderen Spektrum entnommen
m) 270 MHz-Spektrum; $COOCH_3$: 3.72 s
n) $NCOCH_3$: 2.04 s
$COOCH_3$: 3.71 s
o) $NCH_2CN$: 3.60 dd
p) Butyl-$CH_3$: 0.87 t
q) $NCH_2Ph$: ß:3.66 d; α:3.45 d
r) N-Äthyl-$CH_3$: 1.04 t
s) N-Propyl-$CH_3$: 0.84 t
t) treten unter den Meßbedingungen nicht auf
u) 270 MHz-Spektrum, gemessen unter Zusatz von $D_2O$ und $Na_2CO_3$

Tabelle 2, Teil 1:

| Substanz No. Proton | 1[a)] | 2[a)] | 4[a)] | 5[m)] |
|---|---|---|---|---|
| H-1 | 7.90 d | 7.87 d | - | - |
| H-2 | 7.73 t | 7.79 t | 7.35 s | 7.30 s |
| H-3 | 7.35 d | 7.35 d | | |
| H-7 | 5.12 m | 5.00 d | 5.01 d | 5.25 d |
| H-8α | 2.20 m | 1.9-2.2 m | 1.85-2.14 m | |
| H-8ß | | 2.18 dd | 2.19 dd | |
| H-10 | 4.81 s | 4.72 s | 4.73 s | 4.28 s |
| H-13α | 1.7-2.0 m | 1.70 m | 1.69 m | |
| H-13ß | | 1.76 m | 1.77 m | |
| $H_3$-14 | 1.10 t | 1.05 t | 1.06 t | 1.13 t |
| H-1' | 5.43 bs | 5.43 bs | 5.44 d | 5.49 d |
| $H_2$-2' | 1.7-2.0 m | 1.9-2.2 m | 1.85-2.14 m | |
| H-3' | 2.67 m | 3.49 m[h)] | i) | 2.81 bd |
| H-4' | 3.88 bs | 3.86 bs | 3.86 bs | |
| H-5' | 4.10 q | 4.18 q | 4.18 q | 4.10 q |
| $H_3$-6' | 1.35 d | 1.27 d | 1.28 d | 1.37 d |
| N-$CH_3$ | 2.33 s | 2.51 s | 2.52 t | 2.39 bs |
| OH-1 | - | - | 12.28 s | |
| OH-4 | - | 12.12 bs[l)] | | |
| OH-6 | - | 12.88 bs[l)] | 12.99[k)] | |
| OH-11 | - | 13.69 bs[l)] | 13.03[k)] | |
| OH-9 | 3.85 s | 3.63 s | 3.84 s | |

Tabelle 2, Teil 2:

| Substanz No. Proton | 6[c,d,e)] | 7[c,f)] | 8[c,o)] | 9[c,g)] |
|---|---|---|---|---|
| H-1 | 7.88 d; 7.85 d | 7.88 dd | - | 7.87 dd |
| H-2 | 7.72 t; 7.69 t | 7.71 t | 7.28 s | 7.71 t |
| H-3 | 7.33 d; 7.27 d | 7.31 dd | | 7.32 dd |
| H-7 | 5.15 m; 5.12 m | 5.14 m | 5.13 m | 5.17 m |
| H-8α | 2.0-2.6 m | 2.12 dd | 2.12 dd | 2.13 dd |
| H-8ß | | 2.25 d | 2.25 d | 2.28 d |
| H-10 | 4.92 d;4.90 d | 4.94 d | 4.94 s | 4.92 s |
| H-13α | 1.76 m | 1.77 m | 1.77 m | 1.79 m |
| H-13ß | 1.86 m | 1.79-1.94m | 1.80-1.94m | 1.87 m |
| $H_3$-14 | 1.12 t | 1.12 t | 1.13 t | 1.14 t |
| H-1' | 5.55 s; 5.54 s | 5.52 d | 5.52 d | 5.54 bs |
| $H_2$-2' | 2.0-2.6 m | 1.79-1.94m | 1.80-1.94m | 1.91 m |
| H-3' | 3.56 bd; 2.0-2.6m | 2.66 m | 2.68 m | 2.60 dt |
| H-4' | 3.86 d; 3.70 d | 3.70 bs | 3.71 bs | 3.82 bs |
| H-5' | 4.4 q; 4.2 q | 4.16 q | 4.17 q | 4.13 q |
| $H_3$-6' | 1.32 d; 1.30 d | 1.39 d | 1.39 d | 1.43 d |
| N-$CH_3$ | 3.00 s; 2.92 s | 2.42 s | 2.42 s | 2.09 s |
| OH-1 | - | - | 12.26 bs | - |
| OH-4 | 2.08 s; 12.06 s | 12.10 s | | 12.11 bs |
| OH-6 | 12.85 s; 12.82 s | 12.85 s | 12.87 bs | 12.82 bs |
| OH-11 | 13.58 s; 13.52 s | 13.59 s | 12.93 bs | 13.62 bs |
| OH-9 | 3.75 s; 3.66 s | 3.77 s | 3.78 s | |

Tabelle 2, Teil 3:

| Substanz No. Proton | 10[c,q)] | 11[c,r)] | 12[c,s)] | 13[c,p)] |
|---|---|---|---|---|
| H-1 | | 7.87 dd | 7.87 dd | 7.88 dd |
| H-2 | | 7.71 t | 7.71 t | 7.71 t |
| H-3 | | 7.32 dd | 7.31 dd | 7.32 dd |
| H-7 | 5.19 m | 5.15 m | 5.14 m | 5.15 m |
| H-8α | i) | 2.12 dd | 2.12 dd | 2.12 dd |
| H-8ß | 2.28 | 2.25 d | 2.25 d | 2.25 d |
| H-10 | 4.93 | 4.90 d | 4.90 s | 4.91 s |
| H-13α H-13ß | i) | 1.7-1.93 m | 1.7-2.0 m | 1.7-2.0 m |
| $H_3$-14 | 1.13 t | 1.12 t | 1.12 t | 1.12t |
| H-1' | 5.55 bs | 5.53 bd | 5.52 bd | 5.52 d |
| H-2' | i) | i) | i) | 1.7-2.0 m |
| H-3' | 2.65 m | 2.6-2.8 m | 2.59 m | i) |
| H-4' | 3.84 bs | 3.81 bs | 3.77 bs | 3.76 bs |
| H-5' | 4.12 q | 4.09 q | 4.09 q | 4.09 q |
| $H_3$-6' | 1.43 d | 1.39 d | 1.40 d | 1.40 d |
| N-CH3 | 2.13 s | 2.34 s | 2.29 s | 2.29 s |
| OH-1 OH-4 | 12.35 bs | - | - | - |
| OH-4 | | t) | t) | 12.12 bs |
| OH-6 OH-11 | 12.98 bs | t) | t) | 12.82 bs |
| OH-11 | | t) | t) | 13.58 bs |
| OH-9 | i) | t) | t) | i) |

Tabelle 2, Teil 4:

| Substanz No. Proton | 14[c,n] | 15[c] | 17[c] |
|---|---|---|---|
| H-1 | - | 7.91 dd | 7.92 d |
| H-2 | 7.31 t | 7.72 t | 7.72 t |
| H-3 | | 7.32 dd | 7.31 t |
| H-7 | 5.24 bs | 5.51 m | 5.09 m |
| H-8α | 2.1-2.4 m | 2.25 m | i) |
| H-8ß | | | i) |
| H-10 | 4.30 s | 4.99 s | 5.02 s |
| H-13α | 1.47 m | 1.63 m | i) |
| H-13ß | 1.84 m | 1.74 m | i) |
| $H_3$-14 | 1.13 t | 1.12 t | i) |
| H-1' | 5.55 bs | 5.44d; 5.41 d | 5.43 |
| H-2' | | 1.85 m | i) |
| H-3' | | 2.71 m; 2.68 m | i) |
| H-4' | 3.81 bs | 3.60 bs; 3.57 bs | 3.71 bs |
| H-5' | 4.17 q | 4.07 q; 3.93 q | 3.88 q |
| $H_3$-6' | 1.28 d | 1.39 d; 1.33 d | i) |
| N-$CH_3$ | 2.99 s | 2.34 s; 2.33 s | 2.31 s |
| OH-1 | 12.31[k] bs | | - |
| OH-4 | 12.34[k] bs | t) t) | t) |
| OH-6 | 12.84 bs | t) t) | t) |
| OH-11 | 13.00 bs | t) t) | t) |
| OH-9 | | 3.65 bs | t) |

Tabelle 3

400 MHz $^{1}$H-nmr-Daten verschiedener Verbindungen der Formel I gegen Tetramethylsilan als internem Standard

Die Substanz-Nummern der ersten Zeile entsprechen den jeweiligen Beispielsnummern; die Indizes a) bis p) dienen der näheren Charakterisierung:

Abkürzungen:
- s: Singulett
- d: Dublett
- t: Triplett
- q: Quartett
- dd: Dublett eines Dubletts
- bs: breites Singulett

a) N-pentyl-$CH_3$: 0.84 t
b) N-hexyl-$CH_3$: 0.83 t
c) N-heptyl-$CH_3$: 0.84 t
d) N-octyl-$CH_3$ : 0.84 t
e) $NCH_2PhCl$: ß : 3.58 d; α : 3.38 d; Ph : 7.24 d (2H); 7.14 d (2H)
f) $NCH_2PhBr$: ß : 3.56 d; α : 3.36 d; Ph : 7.39 d (2H); 7.08 d (2H)
g) $NCH_2PhNO_2$: ß : 3.70 d; α : 3.56 d; Ph : 8.14 d (2H); 7.40 d (2H)
h) $NCH_2PhCN$: ß : 3.66 d; α : 3.49 d;
i) ausgeschüttelt mit Natriumhydrogencarbonat/Dichlormethan
k) Pentyl-$CH_3$: 0.84 t
l) Ph-$CH_2$ß: 3.65d, 3.64 d; Ph-$CH_2$α: 3.45 d, 3.42 d
m) Pentyl-$CH_3$: 0.84 t; 0.80 t
n) $NCH_2CN$: 3.51
o) $OCH^3$: 4.07 s (3H)
p) 13-Epimerengemisch; die angegebenen Daten beziehen sich auf das überwiegend gebildete Epimer
q) 14-$H_3$ des unterschüssigen 13-Epimers: 1.28 d

Tabelle 3, Teil 1:

| Substanz No. Proton | 18[a)] | 19[b)] | 20[c)] | 21[d)] |
|---|---|---|---|---|
| H-1 | 7.87 dd | 7.86 dd | 7.88 dd | 7.87 dd |
| H-2 | 7.71 t | 7.70 t | 7.71 t | 7.71 t |
| H-3 | 7.32 dd | 7.31 dd | 7.32 dd | 7.32 dd |
| H-7 | 5.14 m | 5.13 m | 5.14 m | 5.14 m |
| H-8α | 2.11 dd | 2.11 dd | 2.13 dd | 2.11 dd |
| H-8ß | 2.26 d | 2.26 d | 2.26 d | 2.26 d |
| H-10 | 4.90 s | 4.90 s | 4.91 s | 4.90 s |
| H-13α H-13ß | 1.72-1.94 m | 1.72-1.94 m | 1.72-1.94 m | 1.72-1.94m |
| $H_3$-14 | 1.12 t | 1.12 t | 1.12 t | 1.12 t |
| H-1' | 5.50 d | 5.49 bs | 5.50 bs | 5.50 bs |
| H-2' H-3' | 1.72-1.94 m | 1.72-1.94 m | 1.72-1.94 m | 1.72-1.94m |
| H-4' | 3.71 bs | 3.67 bs | 3.67 bs | 3.67 bs |
| H-5' | 4.08 q | 4.07 q | 4.07 q | 4.07 q |
| $H_3$-6' | 1.40 d | 1.41 d | 1.41 d | 1.41 d |
| N-$CH_3$ | 2.23 s | 2.18 s | 2.18 s | 2.18 s |
| OH-4 | 12.18 bs | | 12.13 bs | 12.1 bs |
| OH-6 | 12.81 bs | | 12.8 bs | 12.8 bs |
| OH-11 | | | 13.6 bs | 13.5 bs |
| OH-9 | 4.01 bs | 4.05 s | 4.05 s | 4.05 s |

Tabelle 3, Teil 2:

| Substanz No. Proton | 22[e)] | 23[f)] | 24[g)] | 25[h)] |
|---|---|---|---|---|
| H-1 | 7.87 dd | 7.87 dd | 7.88 dd | 7.89 dd |
| H-2 | 7.71 t | 7.71 t | 7.72 dd | 7.72 t |
| H-3 | 7.32 dd | 7.32 dd | 7.32 dd | 7.33 d |
| H-7 | 5.16 m | 5.16 m | 5.17 m | 5.17 m |
| H-8α | 2.13 dd | 2.13 dd | 2.13 dd | 2.14 dd |
| H-8ß | 2.27 d | 2.27 d | 2.27 d | 2.26 d |
| H-10 | 4.92 s | 4.92 s | 4.92 s | 4.92 s |
| H-13α | 1.81 m | 1.79 m | 1.79 m | 1.79 m |
| H-13ß | 1.84-1.98 m | 1.85-1.95 m | 1.84-1.98 m | 1.84-1.98 m |
| $H_3$-14 | 1.13 t | 1.13 t | 1.13 t | 1.13 t |
| H-1' | 5.53 m | 5.53 m | 5.55 bs | 5.54 bs |
| H-2' | 1.84-1.98 m | 1.85-1.95 m | 1.84-1.98 m | 1.84-1.98 m |
| H-3' | 2.60 dt | 2.59 dt | 2.65 dt | 2.63 dt |
| H-4' | 3.80 bs | 3.80 bs | 3.82 bs | 3.80 bs |
| H-5' | 4.12 q | 4.12 q | 4.14 q | 4.13 q |
| $H_3$-6' | 1.42 d | 1.42 d | 1.42 d | 1.42 d |
| N-$CH_3$ | 2.08 s | 2.07 s | 2.12 s | 2.10 s |
| OH-4 | 12.10 bs | 12.10 bs | 12.10 bs | 12.12 bs |
| OH-6 | 12.82 bs | 12.82 bs | 12.83 bs | 12.85 bs |
| OH-11 | 13.60 bs | 13.60 bs | 13.60 bs | 13.61 bs |
| OH-9 | 4.01 bs | 4.01 s | 3.98 s | 3.98 s |

Tabelle 3, Teil 3:

| Substanz No. Proton | 26 [j)] | 27 [i,k)] | 28 [l)] | 29 [m)] |
|---|---|---|---|---|
| H-1 | 7.87 d | | 7.91 dd | 7.91 dd |
| H-2 | 7.71 t | 7.32 s | 7.71 t | 7.72 t |
| H-3 | 7.32 d | | 7.31 dd | 7.32 dd |
| H-7 | 5.14 m | 5.16 m | 5.19 m | 5.15 m |
| H-8α | | | | |
| H-8ß | | | | |
| H-10 | 4.90 s | 4.91 s | 5.03 s | 5.00 s |
| H-13α | | | 1.74 m | |
| H-13ß | | | 1.80-1.94 m | |
| $H_3$-14 | 1.12 t | 1.12 t | 1.13 t | 1.12 t |
| H-1' | 5.50 bs | 5.52 d | 5.54 bs; 5.50 d | 5.48 bs; 5.44d |
| H-2' | | | 1.80-1.94 m | |
| H-3' | | | 2.58-2.70 m | |
| H-4' | 3.66 bs | 3.73 bs | 3.81 bs; 3.76bs | 3.66bs; 3.60bs |
| H-5' | 4.07 q | 4.07 q | 4.10 q; 3.94 q | 4.04 q; 3.90q |
| $H_3$-6' | 1.41 d | 1.40 q | 1.43 d; 1.36 d | 1.39 d; 1.35d |
| N-$CH_3$ | 2.13 s | 2.25 bs | 2.13 s; 2.10 s | 2.17 s; 2.16s |
| OH-1 | | 12.32 bs | | |
| OH-4 | 12.13 bs | | 12.12 bs | 12.14 bs |
| OH-6 | 12.81 bs | 12.92 bs | 12.90 bs | 12.89 bs |
| OH-11 | 13.59 bs | | 13.74 bs | 13.71 bs |
| OH-9 | 4.06 | | | 3.66 bs |

Tabelle 3, Teil 4:

| Substanz No. Proton | 30[n)] | 31[o)] | 32[o,p)] |
|---|---|---|---|
| H-1 | 7.90 dd | 8.02 d | 8.02 d |
| H-2 | 7.71 t | 7.77 t | 7.76 t |
| H-3 | 7.32 dd | 7.38 d | 7.37 t |
| H-7 | 5.16 m | 5.29 m | 5.28 s |
| H-8α | | | |
| H-8ß | | | |
| H-10 | 5.01 s | 3.22 d (10ß) 2.97 d (10α) | 3.19 d (10ß) |
| H-13α | | | |
| H-13ß | | | |
| $H_3$-14 | 1.12 t | 2.41 s | 1.32d[q)] |
| H-1' | 5.49 bd; 5.46 bd | 5.50 bs | 5.51 bd |
| H-2' | | | |
| H-3' | | 2.73 m | |
| H-4' | | 3.62 bs | 3.61 bs |
| H-5' | 4.10 q; 3.96 q | | 3.69 q |
| $H_3$-6' | 1.37 d; 1.34 d | 1.37 d | 1.37 d |
| $N-CH_3$ | 2.40 s; 2.36 s | 2.36 s | 2.34 s |
| OH-4 | 12.14 bs | | |
| OH-6 | 12.89 bs | | |
| OH-11 | 13.75 bs | | |

**Patentansprüche**

**Patentansprüche für folgende Vertragsstaaten : AT, BE, CH, DE, FR, GB, GR, IT, LI, LU, NL, SE**

1. Neue zytostatisch wirksame Anthracyclinderivate der allgemeinen Formel I, welche gegebenenfalls als Salz einer anorganischen oder organischen Säure vorliegen,

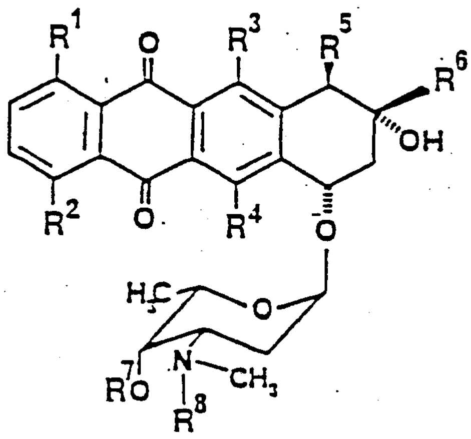

I

in der die Reste folgende Bedeutung haben:

- $R^1$ ist Wasserstoff oder eine Hydroxygruppe,
- $R^2$ ist Wasserstoff, eine Hydroxy- oder eine Methoxygruppe,
- $R^3$ ist Wasserstoff oder eine Hydroxygruppe,
- $R^4$ ist Wasserstoff oder eine Hydroxygruppe,
- $R^5$ ist Wasserstoff, eine Hydroxy-, eine Methoxycarbonylgruppe oder ein Rest der allgemeinen Formel II

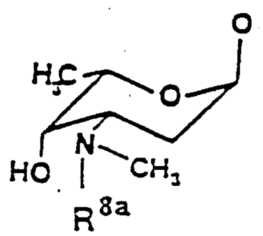

II

in welcher $R^{8a}$ die unter $R^8$ angegbene Bedeutung hat, oder ein Rest der Formel III

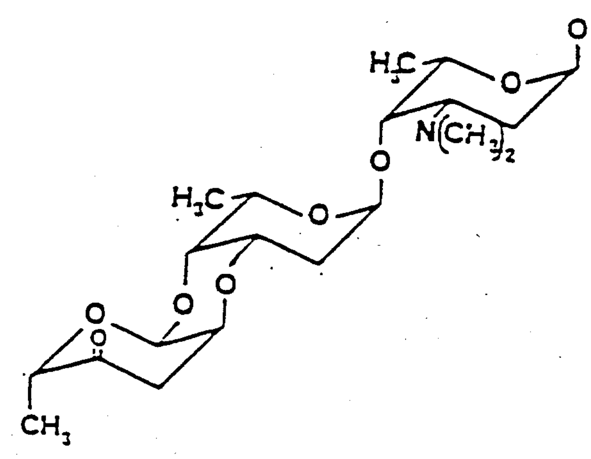

III

- $R^6$ ist Äthyl, Methylcarbonyl, Hydroxymethylcarbonyl, Alkylhydroxy oder Alkyldihydroxy,
- $R^7$ ist Wasserstoff,
- $R^8$ ist Wasserstoff oder eine Cyanomethylgruppe oder ein Substituent der allgemeinen Formel $COR^9$ oder $CH_2R^{10}$, wobei $R^9$ Wasserstoff, $CH_3$, $CF_3$ oder $CCl_3$ und $R^{10}$ $C_1$- bis $C_8$-Alkyl, substituiertes Alkyl, Phenyl oder substituiertes Phenyl ist, ausgenommen die Verbindungen der Formel I, in welcher

  $R^1 = H$, $R^2 = OH$, $R^3 = H$, $R^4 = OH$, $R^5 = COOCH_3$,

$R^6 = CH_2CH_3$ und $R^8 = H$ ist; $R^1 = H$, $R^2 = OCH_3$, $R^3 = R^4 = OH$, $R^5 = H$, $R^6 = COCH_3$ und $R^8 = H$ sowie $R^1 = H$, $R^2 = OCH_3$, $R^3 = R^4 = OH$, $R^5 = H$, $R^6 = COCH_2OH$ und $R^8 = H$ sind, sowie weiterhin für den Fall, daß $R^8$ eine Cyanomethylgruppe ist, diejenigen Verbindungen,in welchen $R^1 = H$ ist, $R^2$ die genannte Bedeutung hat, $R^3 = OH$, $R^4 = OH$, $R^5 = H$ ist, $R^6$ die genannte Bedeutung hat und $R^7 = H$ ist.

**2.** Ahthracyclinderivate nach Anspruch 1 , **dadurch gekennzeichnet,** daß $R^5$ Wasserstoff, eine Hydroxy- oder eine Methoxycarbonylgruppe, $R^8$ Wasserstoff, Cyanomethyl oder ein Substituent der allgemeinen Formel $COR^9$ mit $R^9 = H$, $CH_3$, $CF_3$ oder $CCl_3$ oder ein Substituent der allgemeinen Formel $CH_2R^{10}$ ist, wobei $R^{10}$ $C_1$- bis $C_8$-Alkyl, substituiertes Alkyl, Phenyl oder in ortho-, meta- oder para-Stellung durch Methyl, Äthyl, Hydroxy, Methoxy, Äthoxy, Nitro, Cyano, Fluor, Chlor oder Brom substituiertes Phenyl ist.

**3.** Anthracyclinderivate nach Anspruch 1 , worin $R^1 = H$, $R^2 = R^3 = R^4 = R^5 = OH$ und $R^6 = CH_2CH_3$ ist.

**4.** Anthracyclinderivate nach Anspruch 1 , worin $R^1 = R^2 = R^3 = R^4 = R^5 = OH$ und $R^6 = CH_2CH_3$ ist.

**5.** Anthracyclinderivate nach Anspruch 1, worin $R^1 = H$, $R^2 = OCH_3$, $R^3 = R^4 = OH$,$R^5 = H$ und $R^6 = COCH_3$ ist.

**6.** Anthracyclinderivate nach Anspruch 1 , worin $R^1 = H$, $R^2 = OCH_3$, $R^3 = R^4 = OH$,$R^5 = H$ und $R^6 = COCH_2OH$ ist.

**7.** Anthracyclinderivate nach Anspruch 1, worin $R^1 = H$, $R^2 = OCH_3$, $R^3 = R^4 = OH$,$R^5 = H$ und $R^6 = CH(OH)$-$CH_3$ ist.

**8.** Anthracyclinderivate nach Anspruch 1 , worin $R^1 = H$, $R^2 = OCH_3$, $R^3 = R^4 = OH$,$R^5 = H$ und $R^6 = CH(OH)$-$CH_2OH$ ist.

**9.** Anthracyclinderivate nach Anspruch 1 , worin $R^1 = R^2 = R^3 = R^4 = OH$, $R^5 = COOCH_3$ und $R^6 = CH_2CH_3$ ist.

**10.** Anthracyclinderivate nach Anspruch 1 , worin $R^1 = H$, $R^2 = R^3 = R^4 = OH$, $R^5 = COOCH_3$ und $R^6 = CH_2CH_3$ ist.

**11.** Anthracyclinderivate nach Anspruch 1, worin $R^1 = H$, $R^2 = OH$, $R^3 = H$, $R^4 = OH$, $R^5 = COOCH_3$, $R^6 = CH_2CH_3$, und $R^8$ ungleich $R^7$ ist.

**12.** Anthracyclinderivate nach Anspruch 1 , worin $R^5$ ein Rest der Formel II ist und $R^8$ und $R^{8a}$ identisch sind.

**13.** Anthracyclinderivate nach Anspruch 12 , worin $R^1 = H$, $R^2 = R^3 = R^4 = OH$ und $R^6 = CH_2CH_3$ ist.

**14.** Anthracyclinderivate nach Anspruch 13 , worin $R^1 = OH$, $R^2 = R^3 = R^4 = OH$ und $R^6 = CH_2CH_3$ ist.

**15.** Anthracyclinderivate nach Anspruch 1 , worin $R^5$ ein Rest der Formel III ist.

**16.** Anthracyclinderivate nach Anspruch 15 , worin $R^1 = H$, $R^2 = R^3 = R^4 = OH$ und $R^6 = CH_2CH_3$ ist.

**17.** Anthracyclinderivate nach Anspruch 15 , worin $R^1 = OH$, $R^2 = R^3 = R^4 = OH$ und $R^6 = CH_2CH_3$ ist.

**18.** Verwendung eines Anthracyclinderivates nach Anspruch 1 in einem Arzneimittel.

**Patentansprüche für folgenden Vertragsstaat : ES**

**1.** Verfahren zur Herstellung von zytostatisch wirksamen Anthracyclinderivaten der allgemeinen Formel I, welche gegebenenfalls als Salz einer anorganischen oder organischen Säure vorliegen,

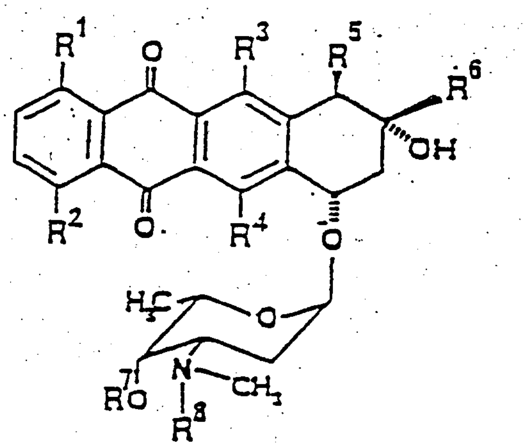

I

in der die Reste folgende Bedeutung haben:

$R^1$ ist Wasserstoff oder eine Hydroxygruppe,

$R^2$ ist Wasserstoff, eine Hydroxy- oder- eine Methoxygruppe,

$R^3$ ist Wasserstoff oder eine Hydroxygruppe,

$R^4$ ist Wasserstoff oder eine Hydroxygruppe,

$R^5$ ist Wasserstoff, eine Hydroxy-, eine Methoxycarbonylgruppe oder ein Rest der allgemeinen Formel II

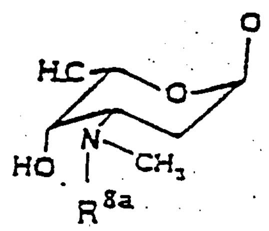

II

in welcher $R^{8a}$ die unter $R^8$ angegebene Bedeutung hat, oder ein Rest der Formel III

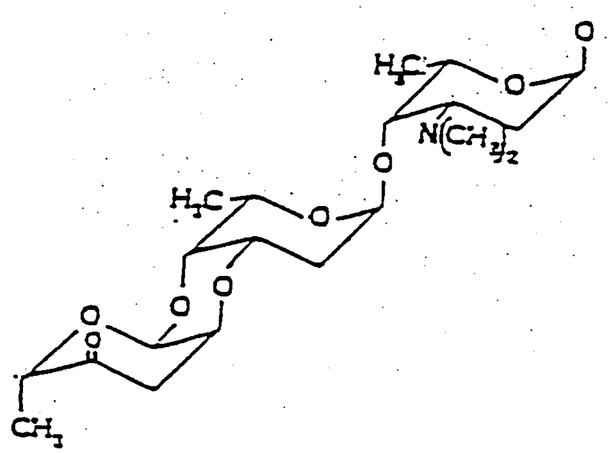

III

$R^6$ ist Äthyl, Methylcarbonyl, Hydroxymethylcarbonyl, Alkylhydroxy oder Alkyldihydroxy,

$R^7$ ist Wasserstoff,

$R^8$ ist Wasserstoff oder eine Cyanomethylgruppe oder ein Substituent der allgemeinen Formel $COR^9$ oder $CH_2R^{10}$, wobei $R^9$ Wasserstoff, $CH_3$, $CF_3$ oder $CCl_3$ und $R^{10}$ $C_1$- bis $C_8$-Alkyl, substituiertes Alkyl, Phenyl oder substituiertes Phenyl ist, ausgenommen die Verbindungen der Formel I, in welcher $R^1 = H$, $R^2 = OH$, $R^3 = H$, $R^4 = OH$, $R^5 = COOCH_3$, $R^6 = CH_2CH_3$ und $R^8 = H$ ist,

$R^1 = H$, $R^2 = OCH_3$, $R^3 = R^4 = OH$, $R^5 = H$, $R^6 = COCH_3$ und $R^8 = H$ sowie $R^1 = H$,

$R^2 = OCH_3$, $R^3 = R^4 = OH$, $R^5 = H$, $R^6 = COCH_2OH$ und $R^8 = H$ sind,
sowie weiterhin für den Fall, daß $R^8$ eine Cyanomethylgruppe ist, diejenigen Verbindungen, in welchen $R^1 = H$ ist, $R^2$ die genannte Bedeutung hat, $R^3 = OH$, $R^4 = OH$, $R^5 = H$ ist, und $R^6$ die genannte Bedeutung hat,
dadurch gekennzeichnet, daß bei einer Verbindung der Formel I, in der die Reste $R^1$ bis $R^7$ die vorstehend angegebene Bedeutung haben und $R^8$ und/oder $R^{8a}$ einen Methylrest darstellen:

a) auf photolytischem Wege unter Lichteinwirkung in Gegenwart eines Lösungsmittelgemisches aus halogeniertem Alkan und Alkohol jeweils eine der beiden am Stickstoff gebundenen Methylgruppen abgespalten wird und

b) gegebenenfalls das in Stufe a) gebildete 3'-N-Methylaminoderivat mit aktiviertem Acetonitril oder gegebenenfalls

c) das in Stufe a) gebildete 3'-N-Methylaminoderivat mit einer aktivierten Acrylverbindung oder gegebenenfalls

d) das in Stufe a) gebildete 3'-N-Methylaminoderivat mit einem Aldehyd oder gegebenenfalls

e) das in Stufe a) gebildete 3'-N-Methylaminoderivat mit einer gegebenenfalls substituierten aliphatischen Jod-, Tosyl- oder Trifluormethansulfonylverbindung

umgesetzt wird und gegebenenfalls

f) das Reaktionsprodukt der Stufe a), b), c), d) oder e) in an sich bekannter Weise in das Salz einer anorganischen oder organischen Säure überführt wird.

2. Verfahren nach Anspruch 1, dadurch gekennzeichnet, daß die photolytische Abspaltung der Methylgruppe in Gegenwart eines Lösungsmittelgemisches aus 10 bis 30 Volumenteilen Chloroform und 1 Volumenteil Alkohol in einer Konzentration zwischen 0,1 und 1 mmol/Liter bei einer Temperatur zwischen 0 °C und der Siedetemperatur des Lösungsmittelgemisches unter starker Lichteinwirkung durchgeführt wird.

3. Verfahren nach Anspruch 1, dadurch gekennzeichnet, daß die photolytische Abspaltung der Methylgruppe in Gegenwart eines Lösungsmittelgemisches aus 10 bis 30 Volumenteilen Chloroform und 1 Volumenteil Methanol in einer Konzentration zwischen 0,1 und 1 mmol/Liter bei einer Temperatur zwischen 0 °C und der Siedetemperatur des Lösungsmittelgemisches unter starker Lichteinwirkung durchgeführt wird.

4. Verfahren zur Herstellung eines Arzneimittels, dadurch gekennzeichnet, daß ein nach Anspruch 1 erhaltenes Anthracyclin-Derivat verwendet wird.

**Claims**

**Claims for the following Contracting States : AT, BE, CH, DE, FR, GB, IT, LI, LU, NL, SE**

1. New anthracycline derivatives having cytostatic activity and the formula I, which are optionally in the form of a salt of an inorganic or organic acid

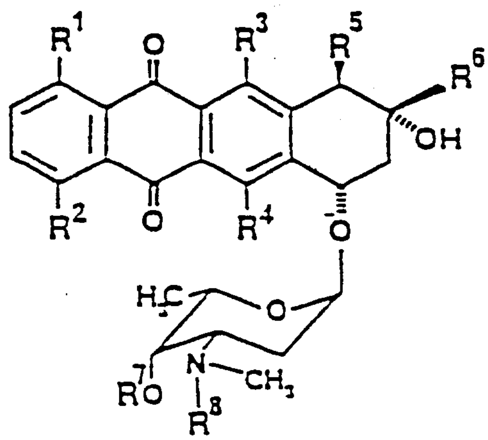

I

in which the radicals have the following meaning:

$R^1$ is hydrogen or a hydroxyl group,
$R^2$ is hydrogen or a hydroxyl or a methoxy group,
$R^3$ is hydrogen or a hydroxyl group,
$R^4$ is hydrogen or a hydroxyl group,
$R^5$ is hydrogen, a hydroxyl or a methoxycarbonyl group, or a radical of the formula II

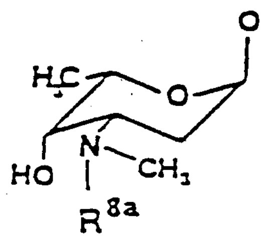

II

in which $R^{8a}$ has the meaning indicated for $R^8$, or a radical of the formula III

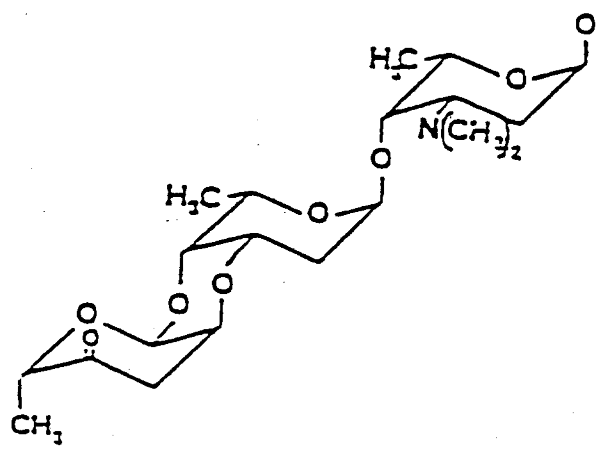

III

$R^6$ is ethyl, methylcarbonyl, hydroxymethylcarbonyl, hydroxyalkyl or dihydroxyalkyl,
$R^7$ is hydrogen,
$R^8$ is hydrogen or a cyanomethyl group or a substituent of the formula $COR^9$ or $CH_2R^{10}$, $R^9$ being hydrogen, $CH_3$, $CF_3$ or $CCl_3$, and $R^{10}$ being $C_1$- to $C_8$-alkyl, substituted alkyl, phenyl or substituted phenyl, excepting the compounds of the formula I in which
$R^1 = H$, $R^2 = OH$, $R^3 = H$, $R^4 = OH$, $R^5 = COOCH_3$, $R^6 = CH_2CH_3$ and $R^8 = H$;
$R^1 = H$, $R^2 = OCH_3$, $R^3 = R^4 = OH$, $R^5 = H$, $R^6 = COCH_3$ and $R^8 = H$, and
$R^1 = H$, $R^2 = OCH_3$, $R^3 = R^4 = OH$, $R^5 = H$, $R^6 = COCH_2OH$ and $R^8 = H$,
and, for the additional case where $R^8$ is a cyanomethyl group, those compounds in which $R^1$ is H, $R^2$ has the said meaning, $R^3$ is OH, $R^4$ is OH, $R^5$ is H, $R^6$ has the said meaning, and $R^7$ is H.

**2.** Anthracycline derivatives as claimed in claim 1, wherein $R^5$ is hydrogen or a hydroxyl or a methoxycarbonyl group, $R^8$ is hydrogen, cyanomethyl or a substituent of the general formula $COR^9$ with $R^9 = H$, $CH_3$, $CF_3$ or $CCl_3$, or a substituent of the formula $CH_2R^{10}$, $R^{10}$ being $C_1$- to $C_8$-alkyl, substituted alkyl, phenyl or phenyl which is substituted in the ortho, meta or para position by methyl, ethyl, hydroxyl, methoxy, ethoxy, nitro, cyano, fluorine, chlorine or bromine.

**3.** Anthracycline derivatives as claimed in claim 1, in which $R^1 = H$, $R^2 = R^3 = R^4 = R^5 = OH$ and $R^6 = CH_2CH_3$.

**4.** Anthracycline derivatives as claimed in claim 1, in which $R^1 = R^2 = R^3 = R^4 = R^5 = OH$ and $R^6 = CH_2CH_3$.

5. Anthracycline derivatives as claimed in claim 1, in which $R^1$ = H, $R^2$ = $OCH_3$, $R^3$ = $R^4$ = OH, $R^5$ = H, $R^6$ = $COCH_3$ and $R^7$ = H.

6. Anthracycline derivatives as claimed in claim 1, in which $R^1$ = H, $R^2$ = $OCH_3$, $R^3$ = $R^4$ = OH, $R^5$ = H and $R^6$ = $COCH_2OH$.

7. Anthracycline derivatives as claimed in claim 1, in which $R^1$ = H, $R^2$ = $OCH_3$, $R^3$ = $R^4$ = OH, $R^5$ = H and $R^6$ = $CH(OH_2)CH_3$.

8. Anthracycline derivatives as claimed in claim 1, in which $R^1$ = H, $R^2$ = $OCH_3$, $R^3$ = $R^4$ = OH, $R^5$ = H and $R^6$ = $CH(OH)CH_2OH$.

9. Anthracycline derivatives as claimed in claim 1, in which $R^1$ = $R^2$ = $R^3$ = $R^4$ = OH, $R^5$ = $COOCH_3$ and $R^6$ = $CH_2CH_3$.

10. Anthracycline derivatives as claimed in claim 1, in which $R^1$ = H, $R^2$ = $R^3$ = $R^4$ = OH, $R^5$ = $COOCH_3$ and $R^6$ = $CH_2CH_3$.

11. Anthracycline derivatives as claimed in claim 1, in which $R^1$ is H, $R^2$ is OH, $R^3$ is H, $R^4$ is OH, $R^5$ is $COOCH_3$, $R^6$ is $CH_2CH_3$, and $R^8$ is not identical to $R^7$.

12. Anthracycline derivatives as claimed in claim 1, in which $R^5$ is a radical of the formula II and $R^8$ and $R^{8a}$ are identical.

13. Anthracycline derivatives as claimed in claim 12, in which $R^1$ = H, $R^2$ = $R^3$ = $R^4$ = OH and $R^6$ - $CH_2CH_3$.

14. Anthracycline derivatives as claimed in claim 13, in which $R^1$ = OH, $R^2$ = $R^3$ = $R^4$ = OH and $R^6$ = $CH_2CH_3$.

15. Anthracycline derivatives as claimed in claim 1, in which $R^5$ is a radical of the formula III.

16. Anthracycline derivatives as claimed in claim 15, in which $R^1$ = H, $R^2$ = $R^3$ = $R^4$ = OH and $R^6$ = $CH_2CH_3$.

17. Anthracycline derivatives as claimed in claim 15, in which $R^1$ = OH, $R^2$ = $R^3$ = $R^4$ = OH and $R^6$ = $CH_2CH_3$.

18. The use of an anthracycline derivative as claimed in claim 1 in a pharmaceutical.

**Claims for the following Contracting State : ES**

1. A process for the preparation of anthracycline derivatives having cytostatic activity and the formula I, which are optionally in the form of a salt of an inorganic or organic acid

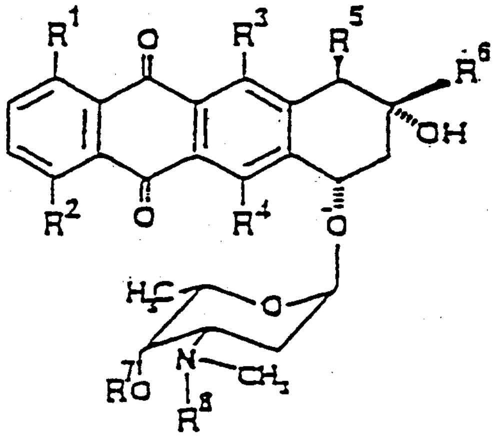

I

in which the radicals have the following meaning:

- $R^1$ is hydrogen or a hydroxyl group,
- $R^2$ is hydrogen or a hydroxyl or a methoxy group,
- $R^3$ is hydrogen or a hydroxyl group,
- $R^4$ is hydrogen or a hydroxyl group,
- $R^5$ is hydrogen, a hydroxyl or a methoxycarbonyl group, or a radical of the formula II

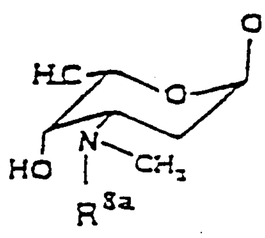

II

in which $R^{8a}$ has the meaning indicated for $R^8$, or a radical of the formula III

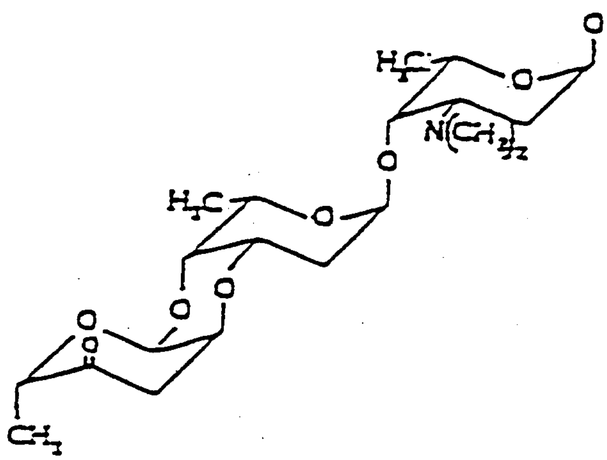

III

- $R^6$ is ethyl, methylcarbonyl, hydroxymethylcarbonyl, hydroxyalkyl or dihydroxyalkyl,
- $R^7$ is hydrogen,
- $R^8$ is hydrogen or a cyanomethyl group or a substituent of the general formula $COR^9$ or $CH_2R^{10}$,$R^9$ being hydrogen, $CH_3$, $CF_3$ or $CCl_3$, and $R^{10}$ being $C_1$- to $C_8$-alkyl, substituted alkyl, phenyl or substituted phenyl, excepting the compounds of the formula I in which
  $R^1 = H$, $R^2 = OH$, $R^3 = H$, $R^4 = OH$, $R^5 = COOCH_3$, $R^6 = CH_2CH_3$ and $R^8 = H$,
  $R^1 = H$, $R^2 = OCH_3$, $R^3 = R^4 = OH$, $R^5 = H$, $R^6 = COCH_3$ and $R^8 = H$, and
  $R^1 = H$, $R^2 = OCH_3$, $R^3 = R^4 = OH$, $R^5 = H$, $R^6 = COCH_2OH$ and $R^8 = H$,

and, for the additional case where $R^8$ is a cyanomethyl group, those compounds in which $R^1$ is H, $R^2$ has the said meaning, $R^3$ is OH, $R^4$ is OH, $R^5$ is H and $R^6$ has the said meaning, which comprises a compound of the formula I in which the radicals $R^1$ to $R^7$ have the meanings indicated above and $R^8$ and/or $R^{8a}$ represent a methyl radical, being subjected to:

a) elimination by photolytic means of a methyl group bonded to nitrogen under the action of light in the presence of a solvent mixture composed of halogenated alkane and an alcohol, and reaction

b) where appropriate of the 3'-N-methylamino derivative formed in stage a) with activated acetonitrile, or where appropriate

c) of the 3'-N-methylamino derivative formed in stage a) with an activated acyl compound, or where appropriate

d) of the 3'-N-methylamino derivative formed in stage a) with an aldehyde, or where appropriate

e) of the 3'N-methylamino derivative formed in stage a) with an optionally substituted aliphatic iodo, tosyl or trifluoromethanesulfonyl compound,

and where appropriate

f) conversion of the reaction product of stage a), b), c), d) or e) in a manner known per se into the salt of an inorganic or organic acid.

**2.** The process as claimed in claim 1, wherein the photolytic elimination of the methyl group is carried out in the presence of a solvent mixture composed of 10 to 30 parts by volume of chloroform and 1 part by volume of alcohol in a concentration between 0.1 and 1 millimole per liter at a temperature between 0 °C and the boiling point of the solvent mixture under the action of intense light.

**3.** The process as claimed in claim 1, wherein the photolytic elimination of the methyl group is carried out in the presence of a solvent mixture composed of 10 to 30 parts by volume of chloroform and 1 part by volume of methanol in a concentration between 0.1 and 1 millimole per liter at a temperature between 0 °C and the boiling point of the solvent mixture under the action of intense light.

**4.** A process for the production of a pharmaceutical, which comprises using an anthracycline derivative obtained as claimed in claim 1.

## Revendications

## Revendications pour les Etats contractants suivants : AT, BE, CH, DE, FR, GB, GR, IT, LI, LU, NL, SE

**1.** Nouveaux dérivés d'anthracycline de formule générale I à activité cytostatique, qui se trouvent éventuellement sous forme de sel d'un acide organique ou minéral,

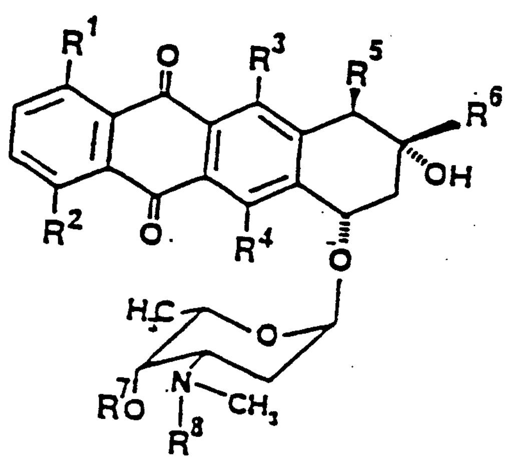

I

formule dans laquelle les radicaux ont les significations suivantes:

$R^1$ est un atome d'hydrogène ou le groupe hydroxy,

$R^2$ est un atome d'hydrogène ou le groupe hydroxy ou méthoxy,

$R^3$ est un atome d'hydrogène ou le groupe hydroxy,

$R^4$ est un atome d'hydrogène ou le groupe hydroxy,

$R^5$ est un atome d'hydrogène, le groupe hydroxy ou méthoxycarbonyle, ou un radical de formule générale II

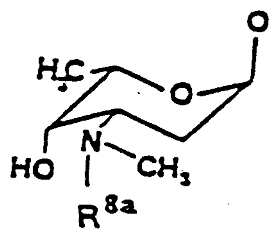

II

dans laquelle $R^{8a}$ a la signification indiquée pour $R^8$, ou un radical de formule III

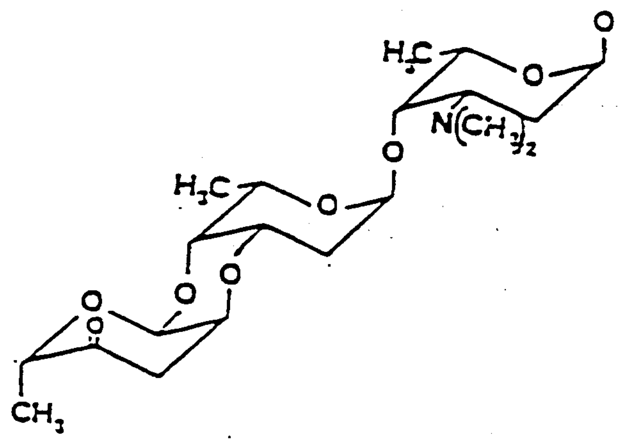

III

$R^6$ est un groupe éthyle, méthylcarbonyle, hydroxyméthylcarbonyle, alkylhydroxy ou alkyldihydroxy,

$R^7$ est un atome d'hydrogène,

$R^8$ est un atome d'hydrogène ou le groupe cyanométhyle ou un substituant de formule générale $COR^9$ ou $CH_2R^{10}$, $R^9$ étant un atome d'hydrogène, $CH_3$, $CF_3$ ou $CCl_3$, et $R^{10}$ étant un groupe alkyle en $C_1$-$C_8$, alkyle substitué, phényle ou phényle substitué,

à l'exception des composés de formule I dans lesquels

$R^1 = H$, $R^2 = OH$, $R^3 = H$, $R^4 = OH$, $R^5 = COOCH_3$, $R^6 = CH_2CH_3$ et $R^8 = H$;

$R^1 = H$ $R^2 = OCH_3$, $R^3 = R^4 = OH$, $R^5 = H$, $R^6 = COCH_3$ et $R^8 = H$ ainsi que

$R^1 = H$, $R^2 = OCH_3$, $R^3 = R^4 = OH$, $R^5 = H$, $R^6 = COCH_2OH$ et $R^8 = H$,

ainsi qu'en outre dans le cas où $R^8$ est le groupe cyanométhyle, les composés dans lesquels $R^1 = H$, $R^2$ a la signification donnée, $R^3 = OH$, $R^4 = OH$, $R^5 = H$, $R^6$ a la signification donnée et $R^7 = H$.

**2.** Dérivés d'anthracycline selon la revendication 1, caractérisés en ce que $R^5$ est un atome d'hydrogène ou le groupe hydroxy ou méthoxycarbonyle, $R^8$ est un atome d'hydrogène, le groupe cyanométhyle ou un substituant de formule générale $COR^9$, dans laquelle $R^9 = H$, $CH_3$, $CF_3$ ou $CCl_3$, ou un substituant de formule générale $CH_2R^{10}$, $R^{10}$ étant un radical alkyle en $C_1$-$C_8$, alkyle substitué, phényle ou un radical phényle substitué en position ortho, méta ou para par un atome de fluor, de chlore ou de brome ou par le groupe méthyle, éthyle, hydroxy, méthoxy, éthoxy, nitro ou cyano.

**3.** Dérivés d'anthracycline selon la revendication 1, dans lesquels $R^1 = H$, $R^2 = R^3 = R^4 = R^5 = OH$ et $R^6 = CH_2CH_3$.

**4.** Dérivés d'anthracycline selon la revendication 1, dans lesquels $R^1 = R^2 = R^3 = R^4 = R^5 = OH$ et $R^6 = CH_2CH_3$.

5. Dérivés d'anthracycline selon la revendication 1, dans lesquels $R^1$ = H, $R^2$ = $OCH_3$, $R^3$ = $R^4$ = OH, $R^5$ = H et $R^6$ = $COCH_3$.

6. Dérivés d'anthracycline selon la revendication 1, dans lesquels $R^1$ = H, $R^2$ = $OCH_3$, $R^3$ = $R^4$ = OH, $R^5$ = H et $R^6$ = $COCH_2OH$.

7. Dérivés d'anthracycline selon la revendication 1, dans lesquels $R^1$ = H, $R^2$ = $OCH_3$, $R^3$ = $R^4$ = OH, $R^5$ = H et $R^6$ = $CH(OH)CH_3$.

8. Dérivés d'anthracycline selon la revendication 1, dans lesquels $R^1$ = H, $R^2$ = $OCH_3$, $R^3$ = $R^4$ = OH, $R^5$ = H et $R^6$ = $CH(OH)CH_2OH$.

9. Dérivés d'anthracycline selon la revendication 1, dans lesquels $R^1$ = $R^2$ = $R^3$ = $R^4$ = OH, $R^5$ = $COOCH_3$ et $R^6$ = $CH_2CH_3$.

10. Dérivés d'anthracycline selon la revendication 1, dans lesquels $R^1$ = H, $R^2$ = $R^3$ = $R^4$ = OH, $R^5$ = $COOCH_3$ et $R^6$ = $CH_2CH_3$.

11. Dérivés d'anthracycline selon la revendication 1, dans lesquels $R^1$ = H, $R^2$ = OH, $R^3$ = H, $R^4$ = OH, $R^5$ = $COOCH_3$, $R^6$ = $CH_2CH_3$ et $R^8$ est différent de $R^7$.

12. Dérivés d'anthracycline selon la revendication 1, dans lesquels $R^5$ est un radical de formule II et $R^8$ et $R^{8a}$ sont identiques.

13. Dérivés d'anthracycline selon la revendication 12, dans lesquels $R^1$ = H, $R^2$ = $R^3$ = $R^4$ = OH et $R^6$ = $CH_2CH_3$.

14. Dérivés d'anthracycline selon la revendication 13, dans lesquels $R^1$ = OH, $R^2$ = $R^3$ = $R^4$ = OH et $R^6$ = $CH_2CH_3$.

15. Dérivés d'anthracycline selon la revendication 1, dans lesquels $R^5$ est un radical de formule III.

16. Dérivés d'anthracycline selon la revendication 15, dans lesquels $R^1$ = H, $R^2$ = $R^3$ = $R^4$ = OH et $R^6$ = $CH_2CH_3$.

17. Dérivés d'anthracycline selon la revendication 15, dans lesquels $R^1$ = OH, $R^2$ = $R^3$ = $R^4$ = OH et $R^6$ = $CH_2CH_3$.

18. Utilisation d'un dérivé d'anthracycline selon la revendication 1 dans un médicament.

**Revendications pour l'Etat contractant suivant : ES**

1. Procédé pour la préparation de dérivés d'anthracycline de formule générale I, à activité cytostatique, qui se trouvent éventuellement sous forme de sel d'un acide organique ou minéral,

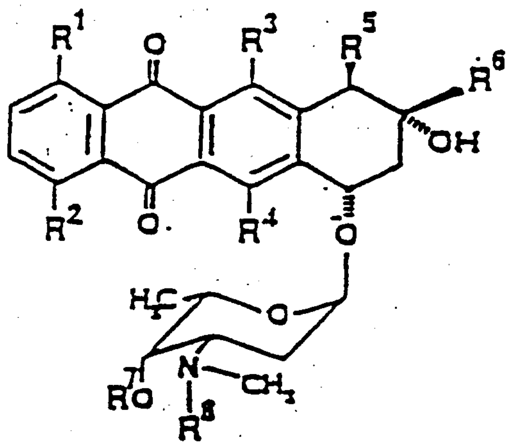

I

formule dans laquelle les radicaux ont les significations suivantes:

- $R^1$ est un atome d'hydrogène ou le groupe hydroxy,
- $R^2$ est un atome d'hydrogène ou le groupe hydroxy ou méthoxy,
- $R^3$ est un atome d'hydrogène ou le groupe hydroxy,
- $R^4$ est un atome d'hydrogène ou le groupe hydroxy,
- $R^5$ est un atome d'hydrogène, le groupe hydroxy ou méthoxycarbonyle, ou un radical de formule générale II

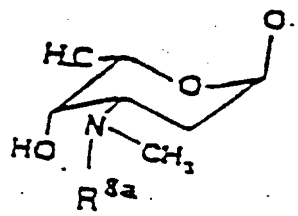

II

dans laquelle $R^{8a}$ a la signification indiquée pour $R^8$, ou un radical de formule III

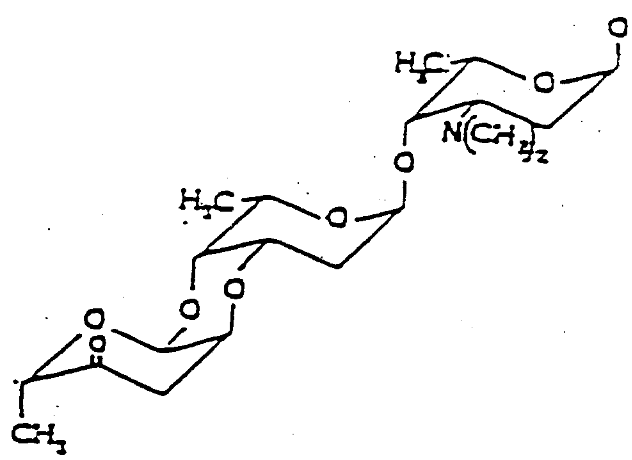

III

- $R^6$ est un groupe éthyle, méthylcarbonyle, hydroxyméthylcarbonyle, alkylhydroxy ou alkyldihydroxy,
- $R^7$ est un atome d'hydrogène,
- $R^8$ est un atome d'hydrogène ou le groupe cyanométhyle ou un substituant de formule générale $COR^9$ ou $CH_2R^{10}$, $R^9$ étant un atome d'hydrogène, $CH_3$, $CF_3$ ou $CCl_3$, et $R^{10}$ étant un groupe alkyle en $C_1$-$C_8$, alkyle substitué, phényle ou phényle substitué,
  à l'exception des composés de formule I dans lesquels
  $R^1$ = H, $R^2$ = OH, $R^3$ = H, $R^4$ = OH, $R^5$ = $COOCH_3$, $R^6$ = $CH_2CH_3$ et $R^8$ = H;

$R^1 = H$, $R^2 = OCH_3$, $R^3 = R^4 = OH$, $R^5 = H$, $R^6 = COCH_3$ et $R^8 = H$ ainsi que
$R^1 = H$ $R^2 = OCH_3$, $R^3 = R^4 = OH$, $R^5 = H$, $R^6 = COCH_2OH$ et $R^8 = H$,
ainsi qu'en outre dans le cas où $R^8$ est le groupe cyanométhyle, les composés dans lesquels $R^1 = H$, $R^2$ a la signification donnée, $R^3 = OH$, $R^4 = OH$, $R^5 = H$ et $R^6$ a la signification donnée,
caractérisé en ce que, dans le cas d'un composé de formule I dans lequel les radicaux $R^1$ à $R^7$ ont les significations données précédemment et $R^8$ et/ou $R^{8a}$ représente(nt) le radical méthyle:

a) on élimine l'un des deux groupes méthyle liés à l'azote, par une voie photolytique, sous l'effet de la lumière, en présence d'un mélange de solvants composé d'un alcane halogéné et d'un alcool, et
b) éventuellement on fait réagir le dérivé 3'-N-méthylamino formé dans l'étape a) avec de l'acétonitrile activé, ou éventuellement
c) on fait réagir le dérivé 3'-N-méthylamino formé dans l'étape a) avec un composé acyle activé, ou éventuellement
d) on fait réagir le dérivé 3'-N-méthylamino formé dans l'étape a) avec un aldéhyde, ou éventuellement
e) on fait réagir le dérivé 3'-N-méthylamino, formé dans l'étape a), avec un composé iodé, tosylé ou trifluorométhanesulfonyle aliphatique éventuellement substitué, et éventuellement
f) on convertit le produit de réaction de l'étape a), b), c), d) ou e), d'une façon connue en soi, en le sel d'un acide organique ou minéral.

**2.** Procédé selon la revendication 1, caractérisé en ce que l'on effectue l'élimination photolytique du groupe méthyle sous l'effet d'une lumière intense, en présence d'un mélange de solvants composé de 10 à 30 parties en volume de chloroforme et de 1 partie en volume d'alcool à une concentration comprise entre 0,1 et 1 mmole/litre, à une température comprise entre 0 °C et le point d'ébullition du mélange de solvants.

**3.** Procédé selon la revendication 1, caractérisé en ce que l'on effectue l'élimination photolytique du groupe méthyle sous l'effet d'une lumière intense, en présence d'un mélange de solvants composé de 10 à 30 parties en volume de chloroforme et de 1 partie en volume de méthanol à une concentration comprise entre 0,1 et 1 mmole/litre, à une température comprise entre 0 °C et le point d'ébullition du mélange de solvants.

**4.** Procédé pour la fabrication d'un médicament, caractérisé en ce qu'on utilise un dérivé d'anthracycline obtenu selon la revendication 1.